# FASCICULE DE BREVET EUROPEEN

(11) **EP 2 168 567 B1**
(45) Date de publication et mention de la délivrance du brevet: **16.12.2015**
(21) Numéro de dépôt: 09164395.7
(22) Date de dépôt: 02.07.2009
(51) Int. Cl.: A61K 8/49, A61Q 17/04, C07D 339/04, A61Q 19/08

(54) **Utilisations de composés dithiolanes pour la photoprotection de la peau, composés dithiolanes et compositions les contenant**
Anwendungen von Dithional-Verbindungen für den Lichtschutz der Haut, Dithional-Verbindungen und sie enthaltende Zusammensetzungen
Uses of dithiolane compounds for the photoprotection of the skin, dithiolane compounds and compositions containing same

(30) Priorité: 24.09.2008 FR 0856414; 28.11.2008 FR 0858078
(43) Date de publication de la demande: 31.03.2010
(73) Titulaire: L'Oréal, 75008 Paris (FR)
(72) Inventeur: Marat, Xavier, 75010 Paris (FR); Lucet-Levannier, Karine, 92500 Reuil-Malmaison (FR); Marrot, Laurent, 91190 LIVRY GARGAN (FR)
(74) Mandataire: Miszputen, Laurent

(56) Documents cités:
- EP-A1- 0 869 126
- WO-A-2008/058999
- DATABASE CA [Online] CHEMICAL ABSTRACTS SERVICE, COLUMBUS, OHIO, US; FUJITA, TAKESHI ET AL: "Dithiolanes as GSH reductase activators" XP002524711 extrait de STN Database accession no. 2000:412186 -& JP 2000 169371 A (SANKYO CO., LTD., JAPAN) 20 juin 2000 (2000-06-20)
- DATABASE CA [Online] CHEMICAL ABSTRACTS SERVICE, COLUMBUS, OHIO, US; FUJI KAGAKU KOGYO K. K.: "Preparation and formulation of cyclic dithio derivatives as remedies for diabetic kidney diseases, hypoglycemic agents, hypolipidemic agents, and lenitives for digestive disorders" XP002524712 extrait de STN Database accession no. 1998:388511 -& EP 0 869 126 A1 (SANKYO CO., LTD., JAPAN) 7 octobre 1998 (1998-10-07)
- BICKERS DAVID R ET AL: "Oxidative stress in the pathogenesis of skin disease" JOURNAL OF INVESTIGATIVE DERMATOLOGY, vol. 126, no. 12, décembre 2006 (2006-12), pages 2565-2575, XP002524710 ISSN: 0022-202X

## Description

La présente invention concerne l'utilisation de composés dithiolanes particuliers de formule (I) dans des compositions utiles dans le domaine du traitement ou de la prévention des troubles induits par un stress oxydatif, notamment provoqués par l'irradiation solaire et à des compositions les contenant.

La peau est l'organe le plus externe de notre corps et est donc la première cible de stress environnementaux, que représentent tout particulièrement les rayonnements ultraviolets de la lumière solaire, UVB et UVA. En effet, l'exposition aigue ou chronique au soleil est connue pour induire des effets biologiques et cliniques délétères sur l'organisme.

Les dommages cutanés liés à une exposition chronique (irradiation répétée) ou aiguë (irradiation forte) aux UV-A ou aux UV-B ont été bien étudiés ; on sait notamment que :
- les UV-B (290-300nm ; 5% des UV totaux), de longueurs d'ondes les plus énergétiques, affectent surtout les cellules épidermiques (kératinocytes), en agissant au niveau de l'ADN ;
- les UV-A (320-400nm ; 95% des UV totaux), plus pénétrantes, atteignent les cellules dermiques telles que les fibroblastes et agissent indirectement via la génération de radicaux libres ;
- de plus, une exposition prolongée aux rayonnements ultraviolets, a pour effet une stimulation de l'expression des collagénases, particulièrement de la métalloprotéinase matricielle de type 1 (MMP-1).

Sur les plans cellulaire et moléculaire, l'impact des rayonnements UVB et UVA induit diverses réactions, dont l'induction directe et indirecte de lésions à l'ADN.

Parmi l'induction directe de lésions à l'ADN, certaines sont spécifiques des rayonnements UV, comme les dimères de pyrimidines et les 6,4 photoproduits. En cas d'erreur lors de la réparation par les systèmes enzymatiques spécialisés (nucléotide excision repair NER, ou global excision repair GER), elles peuvent être responsables de mutations elles-mêmes à l'origine de processus tumoral aboutissant au développement de cancers cutanés. On retrouve d'ailleurs dans les cellules issues de ces tumeurs une incidence très élevée de mutations caractéristiques de l'impact des UV solaires. Ces lésions à l'ADN sont aussi à l'origine de processus apoptotiques induisant la formation de cellules caractéristiques dans l'épiderme que sont les « sunburn cells ». On notera aussi que les UV sont responsables sur le plan cellulaire de la génération d'espèces réactives de l'oxygène, elles-mêmes à l'origine de nombreux effets biologiques comme l'induction de dommages oxydatifs à l'ADN (8 oxo Guanine) ou de nombreux gènes.

Enfin en plus des effets décrits principalement sur les deux types cellulaires majeurs de la peau que sont les kératinocytes qui forment l'épiderme stratifié et différencié, et les fibroblastes responsables de la synthèse et du renouvellement de la matrice extracellulaire dermique, les UV ont aussi un impact sur les cellules de Langerhans, ayant une fonction immunitaire de présentation de l'antigène.

Les effets délétères des UV dans la peau (érythème, photocarcinogénèse, photovieillissement, photo-immunosuppression....) sont induits par une action directe des UV sur certains chromophores cellulaires comme l'ADN, mais aussi par une action indirecte. L'énergie transportée par les UV est en effet capable de déclencher la formation d'espèces activées de l'oxygène (EAO), comme l'oxygène singulet et l'anion superoxyde, par le biais de réaction de photosensibilisation impliquant des photosensibilisants endogènes tels que les riboflavines, les bilirubines, la phaéomélanine et les dérivés porphyriniques. L'oxygène singulet et l'anion superoxyde vont faire l'objet d'une cascade de réactions entraînant la production d'autres EAO comme le peroxyde d'hydrogène et les radicaux hydroxyles. Les EAO ainsi générés, endommagent l'ADN, les membranes cellulaires et certaines protéines (enzymes, facteurs de transcription...).

Les cellules sont équipées d'une défense antioxydante enzymatique (superoxides dismutases Cu-Zn et Mn, catalases, glutathion peroxydases...) et non enzymatique (vitamines E et C, thiols dont le glutathion, β-carotène, oligo-éléments...) dont le rôle est de maintenir le potentiel redox intracellulaire, mais cette capacité de défense peut être dépassée lors d'un stress oxydatif intense.

Le tripeptide glutathion (γ-L-glutamyl-L-cysteinyl-glycine ou GSH) est le plus ubiquitaire et abondant des thiols non protéiniques de faible poids moléculaire. La majeure partie du GSH intracellulaire se trouve sous forme réduite (GSH). Le glutathione-disulfure (GSSG) représente moins de 0.5% du GSH total. Dans la plupart des cellules animales, la concentration du GSH est comprise entre 1 et 10mM alors qu'elle est de l'ordre de 0.5 et 10µM dans le plasma. La fonction thiol localisée sur le résidu cystéine lui confère un potentiel redox (environ -230 mV) prépondérant dans les phénomènes métaboliques d'oxydo-réduction. Ses propriétés réductrices et nucléophiles jouent un rôle majeur dans la protection contre les altérations oxydantes des lipides, des protéines et des acides nucléiques. En situation de stress oxydant, son rôle protecteur et détoxifiant résulte principalement de sa fonction coenzyme des glutathion peroxydases et des glutathion-S-transférases. Il fait aussi l'objet d'interactions synergiques avec d'autres composants du système de protection antioxydante tels que la vitamine C, la vitamine E, et les superoxyde dismutases.

La diminution du niveau de glutathion va donc affecter la balance cellulaire redox. Il est notamment connu qu'une exposition aux UV entraîne la déplétion du taux de GSH intracellulaire augmentant ainsi la sensibilité des cellules face au stress oxydatif.

La peau peut être protégée des effets néfastes du rayonnement UV par l'utilisation de filtres solaires. Ces produits contiennent des molécules absorbant les longueurs d'onde nocives avant qu'elles n'atteignent la peau et l'endommagent, prévenant ainsi des effets aigus et chroniques d'une exposition aux UV.

Pourtant les filtres solaires n'ont pas une action globale. Dés lors qu'il n'existe pas de filtres permettant une absorption totale des longueurs d'ondes nocives (UVB, UVA et UVA long), une stratégie de photoprotection basée sur l'induction des systèmes de défense antioxydante endogène, offre des perspectives intéressantes.

Il existe donc un réel besoin de trouver des solutions complémentaires dans ce domaine afin de reconstituer et/ou préserver le taux de glutathion endogènes après exposition aux UV. Ceci peut être envisagé par la stimulation par un actif des systèmes naturels endogènes de défense et/ou de réparation cellulaire après un stress UV.

L'acide lipoïque ou thioctique (1,2 dithiacyclopentane-3-valérique) est un dithiol endogène largement trouvé dans les plantes et les animaux. C'est une coenzyme du métabolisme des lipides et des hydrates de carbone dans les complexes multienzymatiques mitochondriaux tels que la pyruvate déshydrogénase et l'α-ketoglutarate déshydrogénase. L'acide lipoïque accroît également le taux cellulaire de glutathion par régénération du glutathion oxydé (GSSG) et l'augmentation de l'activité de γ-glutamylcysteine ligase (enzyme contrôlant la synthèse de GSH).

On connaît dans les demandes EP0869126 et JP2000169371 des composés dithiolanes améliorant l'activité de la glutathion reductase, pour le traitement ou la prévention des troubles induits par un stress oxydatif.

On connaît dans la demande WO2008/058999 des composés dithiolanes siloxaniques ou silaniques permettent d'augmenter le taux de glutathion après déplétion UV induite notamment le composé 5-(1,2-dithiolan-3-yl)-N-[3-(triméthylsilyl)propyl]pentanamide et le composé 5-(1,2-dithiolan-3-yl)pentanoate de (triméthylsilyl)méthyle. Cependant la protection apportée par ces actifs vis-à-vis de la déplétion du GSH induite par les UV Journaliers n'est pas encore pleinement satisfaisante.

La Demanderesse a maintenant découvert que certains composés dithiolanes de formules (I) particulières que l'on définira en détail ci-dessous permettent d'augmenter de manière significative le taux de glutathion après déplétion UV induite par rapport aux composés dithiolanes de l'art antérieur et de pouvoir ainsi de renforcer et/ou préserver la protection antioxydante naturelle de la peau face au stress oxydatif notamment causé par les radiations UV.

La présente invention a donc pour objet l'utilisation cosmétique non thérapeutique d'au moins un composé dithiolane de formule (I) dans une composition comprenant un milieu physiologiquement acceptable, dans le but de renforcer et/ou de préserver la protection antioxydante naturelle de la peau, notamment le système de défense antioxydant endogène face au stress oxydatif notamment causé par les radiations UV.

La présente invention a en particulier pour objet l'utilisation cosmétique d'au moins un composé dithiolane de formule (I) dans une composition comprenant un milieu physiologiquement acceptable, dans le but de renforcer et/ou de préserver le taux de glutathion intracellulaire endogène apportant la protection antioxydante naturelle de la peau.

Par « peau », on entend toute surface cutanée du corps humain, y compris peau, muqueuse, semi-muqueuse, incluant donc lèvres, cuir chevelu ainsi que les annexes de la peau, notamment ongles, poils, cheveux.

Un « milieu physiologiquement acceptable » est selon l'invention soit un milieu cosmétiquement ou pharmaceutiquement acceptable compatible avec la peau, les muqueuses, les ongles et/ou les cheveux, soit un milieu administrable par la voie orale.

D'autres objets de l'invention seront définis plus loin dans la suite de la description.

Les composés dithiolanes conformes à la présente invention répondent à la formule (I) suivante : 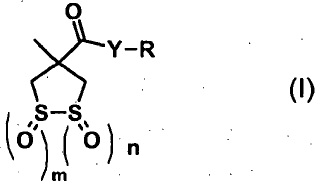 dans laquelle :
Y désigne O, NR₁ ou S
R₁ désigne un atome d'hydrogène ; un groupe hydrocarboné alkyle saturé linéaire en C₁-C₂₀ ou ramifié en C₃-C₂₀ ou insaturé en C₂-C₂₀ ; un groupe phényle éventuellement substitué par un ou plusieurs hydroxyles et/ou par un ou plusieurs radicaux alkoxy en C₁-C₈ ;
R désigne un atome d'hydrogène ; ou un groupe hydrocarboné alkyle saturé linéaire en C₁-C₂₀ ou ramifié en C₃-C₂₀ ou insaturé en C₂-C₂₀ ; ou un groupe phényle éventuellement substitué par un ou plusieurs hydroxyles et/ou par un ou plusieurs radicaux alkoxy en C₁-C₈ ; ou un groupe alkyle saturé en C₁-C₈ possédant un substituant phényle éventuellement substitué par un ou plusieurs hydroxyles et/ou par un ou plusieurs radicaux alcoxy en C₁-C₈ ;.
R possède éventuellement un ou plusieurs substituants choisis parmi OR₂, SR₂, NR₂R₃, COOR₂ dans lesquels :
   R₂ désigne un atome d'hydrogène ou un groupe hydrocarboné saturé linéaire en C₁-C₅ ou ramifié en C₃-C₅ ou insaturé en C₂-C₅, ou un groupe phényle ;
   R₃ désigne un atome d'hydrogène ; un groupe hydrocarboné saturé linéaire en C₁-C₅ ou ramifié en C₃-C₅ ou insaturé en C₂-C₅, ; un groupe phényle; un groupe acétyle. Lorsque Y désigne NR₁, R et R₁ peuvent former un cycle choisi parmi pyrrolidine, pyrroline, pipéridine, pipérazine, morpholine, thiomorpholine, azépine m = 0 ou 1 ou 2
   n = 0 ou 1 ou 2
ainsi que leurs sels, leurs chélates, leurs solvates et leurs isomères optiques.

Les sels des composés décrits dans la présente invention comprennent des sels non toxiques conventionnels desdits composés tels que ceux formés à partir d'acides organiques ou minéraux. A titre d'exemple, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique. Les sels de bases organiques ou minérales telles que les sels de triéthanolamine, d'aminopropanediol, de sodium, de zinc.

Les solvates acceptables pour l'usage non thérapeutique des composés décrits dans la présente invention comprennent des solvates conventionnels tels que ceux formés lors de la dernière étape de préparation desdits composés du fait dé la présence de solvants. A titre d'exemple, on peut citer les solvates dus à la présence d'eau ou d'alcools linéaires ou ramifiés comme l'éthanol ou l'isopropanol.

Les isomères optiques sont notamment les énantiomères et les diastéréoismères.

De façon préférentielle, les groupes alcoxy sont linéaires en C₁-C₄ et plus préférentiellement méthoxy, éthoxy, propoxy, butoxy et encore plus préférentiellement méthoxy.

De façon préférentielle, les groupes hydrocarbonés sont des alkyles linéaires ou ramifiés et peuvent être choisis parmi : méthyle, éthyle, propyle, isopropyle, butyle, isobutyle, tertio-butyle pentyle, hexyle, heptyle octyle, nonyle, décyle, undécyle, docécyle, tridecyle, tetradecyle, pentadécyle, hexadécyle, heptadécyle, octadécyle, nonadecyle, icosyle.

De façon plus préférentielle, les groupes hydrocarbonés sont des radicaux alkyles linéaires ou ramifiés saturés en C₁-C₈ : méthyle, éthyle propyle isopropyle butyle isobutyle, tertio-butyle, pentyle, hexyle, heptyle, octyle.

De préférence, les composés de formule (I) ont les significations suivantes :
Y désigne S, O, NR₁
R₁ désigne un atome d'hydrogène ; un groupe hydrocarboné alkyle saturé linéaire en C₁-C₁0 ou ramifié en C₃-C₁₀ ;
R désigne un atome d'hydrogène ; un groupe hydrocarboné alkyle saturé linéaire en C₁-C₂₀ ou ramifié en C₃-C₂₀ ; un groupe phényle éventuellement substitué par un ou plusieurs hydroxyles et/ou par un ou plusieurs radicaux alcoxy en C₁-C₃ ; un groupe alkyle saturé en C₁-C₅ possédant un substituant phényle éventuellement substitué par un ou plusieurs hydroxyles et/ou par un ou plusieurs radicaux alcoxy en C₁-C₃ ; un groupe hydrocarboné alkyle linéaire en C₁-C₅ substitué par un ou plusieurs groupes identiques ou non choisis parmi OR₂, SR₂, NR₂R₃, COOR₂ dans lesquels:
   R₂ désigne un atome d'hydrogène ou un groupe hydrocarboné saturé linéaire en C₁-C₅ ou ramifié en C₃-C₅ ou insaturé en C₂-C₅,
   R₃ désigne un atome d'hydrogène ; un groupe hydrocarboné saturé linéaire en C₁-C₅ ou ramifié en C₃-C₅ ; un groupe phényle ; un groupe acétyle ;
   lorsque Y=NR1, R et R₁ peuvent former un cycle pyrrolidine,
   n = 0 ou 1 ou 2
   m = 0 ou 1 ou 2
ainsi que leurs sels d'acide ou de base, leurs chélates, leurs solvates et leurs isomères optiques.

Plus préférentiellement, les composés de formule (I) ont les significations suivantes :
Y désigne O ou NR₁ ;
R₁ désigne un atome d'hydrogène ; un groupe hydrocarboné alkyle saturé linéaire en C₁-C₁₀ ou ramifié en C₃-C₁₀;
R désigne un atome d'hydrogène ; un groupe hydrocarboné alkyle saturé linéaire en C₁-C₁₀ ou ramifié en C₃-C₁₀ ; un groupe phényle éventuellement substitué par un ou plusieurs hydroxyles et/ou par un ou plusieurs radicaux méthoxy ; un groupe hydrocarboné alkyle saturé en C₁-C₃ possédant un substituant phényle éventuellement substitué par un ou plusieurs hydroxyles et/ou par un ou plusieurs radicaux méthoxy ; un groupe hydrocarboné alkyle linéaire en C₁-C₄ substitué par un ou plusieurs groupes identiques ou non choisis parmi OR₂, SR₂; NR₂R₃, COOR₂ dans lesquels :
   R₂ désigne un atome d'hydrogène ou un groupe hydrocarboné saturé linéaire en C₁-C₅ ou ramifié en C₃-C₅,
   R₃ désigne un atome d'hydrogène ; un groupe hydrocarboné saturé linéaire en C₁-C₅ ou ramifié en C₃-C₅;
   n = 0 ou 1 ou 2
   m = 0 ou 1 ou 2
ainsi que leurs sels d'acide ou de base, leurs chélates, leurs solvates et leurs isomères optiques.

Encore plus préférentiellement, les composés de formule (I) ont les significations suivantes :
Y désigne NR₁
R₁ désigne un atome d'hydrogène ; un groupe hydrocarboné allyle saturé linéaire en C₁-C₄ ;
R désigne un atome d'hydrogène ; un groupe hydrocarboné alkyle saturé linéaire en C₁-C₁₀ ou ramifié en C₃-C₁₀ ; un groupe phényle ; un groupe alkyle saturé linéaire en C₁-C4 substitué par un phényle substitué ou non par un ou plusieurs groupes identiques ou non choisis parmi OH, OMe ; un groupe hydrocarboné alkyle linéaire en C1-C4 substitué par un ou plusieurs groupes identiques ou non choisis parmi OH, NHAc, SR₂, COOR₂ avec R₂ étant un hydrogène ou un groupe alkyle linéaire en C₁-C₄ ;
n = 0 ou 1 ou 2 , m = 0 ou 1 ou 2
ainsi que leurs sels d'acide ou de base, leurs chélates, leurs solvates et leurs isomères optiques.

Plus particulièrement, les composés de formule (I) ont les significations suivantes :
Y désigne NH
R désigne un atome d'hydrogène ; un groupe hydrocarboné alkyle saturé linéaire en C₁-C₁₀ ou ramifié en C₃-C₁₀ ; un groupe phényle ; un groupe alkyle saturé linéaire en C₁-C₄ substitué par un phényle substitué ou non par un ou plusieurs groupes identiques ou non choisis parmi OH, OMe ; un groupe hydrocarboné alkyle linéaire en C₁-C₄ substitué par un ou plusieurs groupes identiques ou non choisis parmi OH, NHAc, SR₂, COOR₂ avec R₂ étant un hydrogène ou un groupe alkyle linéaire en C₁-C₄ ;
n = 0 ou 1 ou 2 ;
m = 0 ou 1 ou 2 ;
ainsi que leurs sels d'acide ou de base, leurs chélates, leurs solvates et leurs isomères optiques.

Encore plus particulièrement, les composés de formule (I) ont les significations suivantes :
Y désigne NH
R désigne un atome d'hydrogène ; un groupe hydrocarboné alkyle saturé linéaire en C₁-C₁₀ ou ramifié en C₃-C₁₀ ;
n = 0 ou 1 ou 2 ;
m = 0 ou 1 ou 2 ;
ainsi que leurs sels d'acide ou de base, leurs chélates, leurs solvates et leurs isomères optiques.

De façon préférentielle, Y = O, NR1.

De façon plus préférentielle, Y = NR1.

De façon encore plus préférentielle, Y = NH.

De façon très préférentielle, R = H ou un radical alkyle en C1-C8.

Parmi les composés de formule (I), on utilisera de préférence les composés suivants

| **N°** | **Structure** | **Nom Chimique** |
|---|---|---|
| 1 | 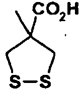 | Acide 4-methyl-1,2-dithiolane-4-carboxylique |
| 2 | 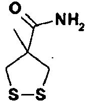 | 4-methyl-1,2-dithiolane-4-carboxamide |
| 3 | 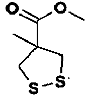 | Méthyl 4-methyl-1,2-dithiolane-4-carboxylate |
| 4 | 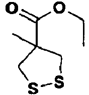 | Ethyl 4-methyl-1,2-dithiolane-4-carboxylate |
| 5 | 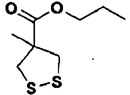 | Propyl 4-methyl-1,2-dithiolane-4-carboxylate |
| 6 | 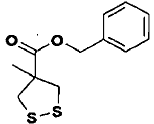 | Benzyl 4-methyl-1,2-dithiolane-4-carboxylate |
| 7 | 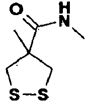 | N-methyl 4-methyl-1,2-dithiolane-4-carboxamide |
| 8 | 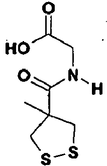 | {[(4-methyl-1,2-dithiolan-4-yl)carbonyl]amino} acetic acide |
| 9 | 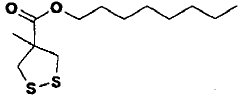 | Octyl 4-methyl-1,2-dithiolane-4-carboxylate |

| | | |
|---|---|---|
| 10 | 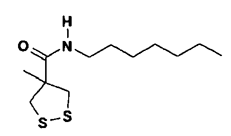 | N-heptyl-4-methyl-1,2-dithiolane-4-carboxamide |
| 11 | 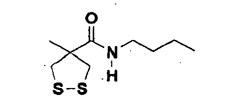 | N-butyl-4-methyl-1,2-dithiolane-4-carboxamide |
| 12 | 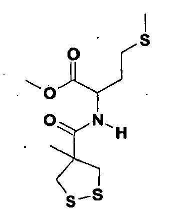 | Methyl 2-{[(4-methyl-1,2-dithiolan-4-yl)carbonyl]amino}-4-(methylsulfanyl)butanoate |
| 13 | 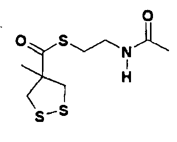 | S-[2-(acetylam ino)ethyl] 4-methyl-1,2-dithiolane-4-carbothioate |
| 14 | 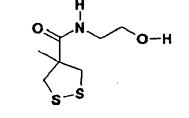 | N-(2-hydroxyethyl)-4-methyl-1,2-dithiolane-4-carboxamide |
| 15 | 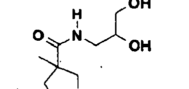 | N-(2,3-dihydroxypropyl)-4-methyl-1,2-dithiolane-4-carboxamide |
| 16 | 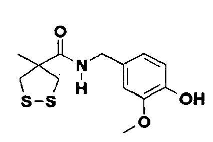 | N-(4-hydroxy-3-methoxybenzyl)-4-methyl-1,2-dithiolane-4-carboxamide |
| 17 | 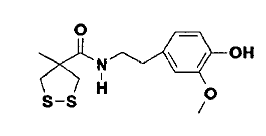 | N-[2-(4-hydroxy-3-methoxyphenyl)ethyl]-4-methyl-1,2-dithiolane-4-carboxamide |
| 18 | 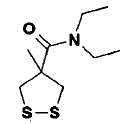 | N,N-diethyl-4-methyl-1,2-dithiolane-4-carboxamide |
| 19 | 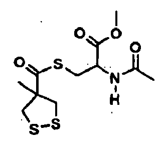 | Methyl 2-(acetylamino)-3-{[(4-methyl-1,2-dithiolan-4-yl)carbonyl]sulfanyl}propanoate |
| 20 | 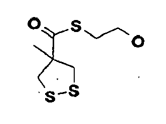 | S-(2-hydroxyethyl) 4-methyl-1,2-dithiolane-4-carbothioate |
| 21 | 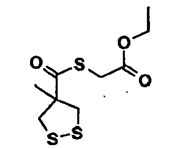 | Ethyl {[(4-methyl-1,2-dithiolan-4-yl)carbonyl]sulfanyl}acetate |
| 22 | 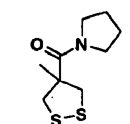 | [(4-methyl-1,2-dithiolan-4-yl)carbonyl]pyrrolidine |
| 23 | 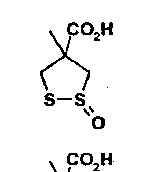 | 4-methyl-1,2-dithiolane-4-carboxylic acid 1-oxide |
| 24 | 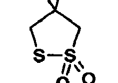 | 4-methyl-1,2-dithiolane-4-carboxylic acid 1,1-dioxide |
| 25 | 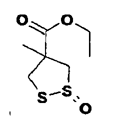 | Ethyl 4-methyl-1,2-dithiolane-4-carboxylate 1-oxide |
| 26 | 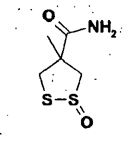 | 4-methyl-1,2-dithiolane-4-carboxamide 1-oxide |
| 27 | 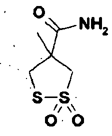 | 4-methyl-1,2-dithiolane-4-carboxamide 1,1-dioxide |
| 28 | 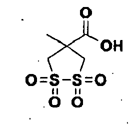 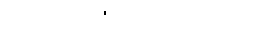 | 4-methyl-1,2-dithiolane-4-carboxylic acid 1,1,2,2-tetraoxide |
| 29 | 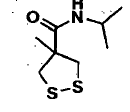 | 4-methyl-N-(1-methylethyl)-1,2-dithiolane-4-carboxamide |
| 30 | 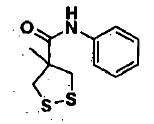 | 4-methyl-N-phenyl-1,2-dithiolane-4-carboxamide |
| 31 | 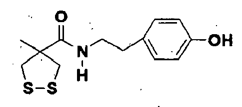 | N-[2-(4-hydroxyphenyl)ethyl]-4-methyl-1,2-dithiolane-4-carboxamide |
| 32 | 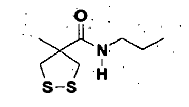 | N-propyl-4-methyl-1,2-dithiolane-4-carboxamide |
| 33 | 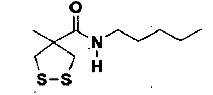 | N-pentyl-4-methyl-1,2-dithiolane-4-carboxamide |
| 34 | 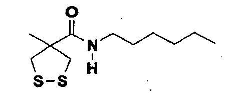 | N-hexyl-4-methyl-1,2-dithiolane-4-carboxamide |
| 35 | 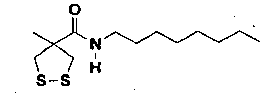 | N-octyl-4-methyl-1,2-dithiolane-4-carboxamide |
| 36 | 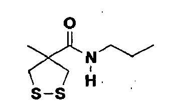 | N-propyl-4-methyl-1,2-dithiolane-4-carboxamide |
| 37 | 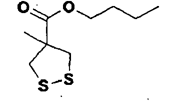 | Butyl 4-methyl-1,2-dithiolane-4-carboxylate |
| 38 | 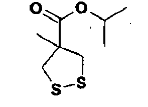 | Isopropyl 4-methyl- 1,2 -dithiolane-4-carboxylate |
| 39 | 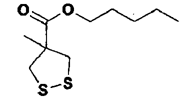 | pentyl 4-methyl-1,2-dithiolane-4-carboxylate |
| 40 | 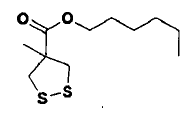 | hexyl 4-methyl-1,2-dithiolane-4-carboxylate |
| 41 | 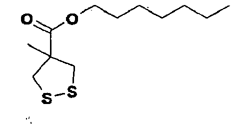 | heptyl 4-methyl-1,2-dithiolane-4-carboxylate |

Parmi ces composés, on préfère plus particulièrement les composés suivants :

| **N°** | **Structure** | **Nom Chimique** |
|---|---|---|
| 1 | 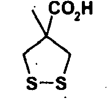 | Acide 4-methyl-1,2-dithiolane-4-carboxylique |
| 2 | 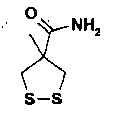 | 4-methyl-1,2-dithiolane-4-carboxamide |
| 9 | 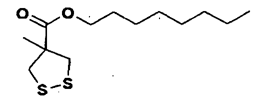 | Octyl 4-methyl-1,2-dithiolane-4-carboxylate |
| 10 | 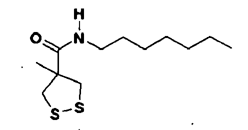 | N-heptyl-4-methyl-1,2-dithiolane-4-carboxamide |
| 26 | 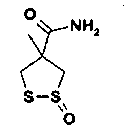 | 4-methyl-1,2-dithiolane-4-carboxamide 1-oxide |
| 27 | 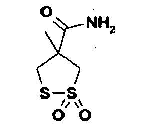 | 4-methyl-1,2-dithiolane-4-carboxamide 1,1-dioxide |

Certains des composés conformes à l'invention sont connus en soi. Il s'agit des composés 1 à 8 suivants :

| **N°** | **Structure** | **Nom Chimique** | **CAS** |
|---|---|---|---|
| 1 | 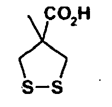 | Acide 4-methyl-1,2-dithiolane-4-carboxylique | 208243-72-5 |
| 2 | 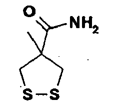 | 4-methyl-1,2-dithiolane-4-carboxamide | 208243-73-6 |
| 3 | 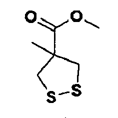 | Méthyl 4-methyl-1,2-dithiolane-4-carboxylate | 208243-88-3 |
| 4 | 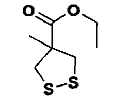 | Ethyl 4-methyl-1,2-dithiolane-4-carboxylate | 208243-89-4 |
| 5 | 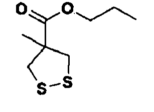 | Propyl 4-methyl-1,2-dithiolane-4-carboxylate | 208243-90-7 |
| 6 | 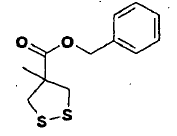 | Benzyl 4-methyl-1,2-dithiolane-4-carboxylate | 208243-73-6 |
| 7 | 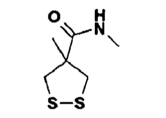 | N-methyl 4-methyl-1,2-dithiolane-4-carboxamide | 208243-91-8 |
| 8 | 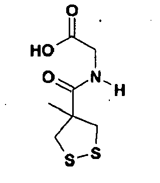 | {[(4-methyl-1,2-dithiolan-4-yl)carbonyl]amino} acetic acide | 208243-74-7 |

Ces derniers ont été décrits dans la demande WO98/23606 et utilisés en pharmacologie comme agents pour réduire le glucose ou les lipides dans le sang

Les composés de formule (I) à l'exception des composés 1 à 8 sont nouveaux et constituent un autre objet de l'invention.

Un autre objet de l'invention consiste en des compositions cosmétiques ou pharmaceutiques comprenant dans un milieu physiologiquement acceptable au moins un composé de formule (I) à l'exception des composés 1 à 8 tels que définis ci-dessus.

### Synthèse

Les composés de formule (I) peuvent être obtenus suivant les voies décrites ci-dessous et documentées
- dans la revue de Lene Teuber, Sulfur reports, 9(4), 257-349,1990 Naturally occuring 1,2-dithiolanes and 1,2,3-tritianes. Chemical and Biological Properties.
- dans la demande de brevet EP 0 869 126 A1.

A partir de l'acide 2,2 bis(hydroxymethyl)propionique (CAS : 4767-03-7), par fonctionnalisation des hydroxyles en groupements partants X (alkyle ou aryles sulfonates tels que mésylates ou tosylates ou halogènes tels que l'iode, le brome ou le chlore) suivie de l'introduction du soufre selon le schéma réactionnel suivant :

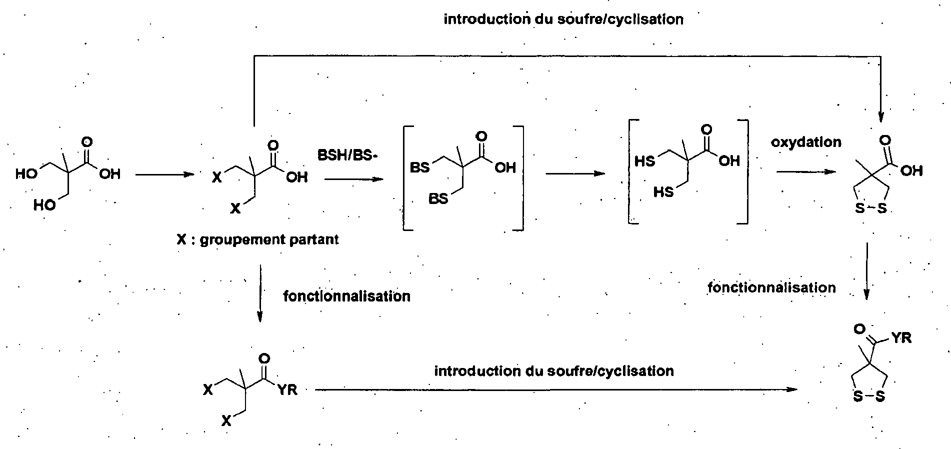

Cette introduction du souffre peut s'effectuer :
(i) en une étape à l'aide de disulfure de métaux (comme le Na₂S₂) ou des sels de tétrathiomolybdates dans des solvants polaires protiques ou aprotiques (par exemple eau, DMF, méthanol, acétonitrile) pour conduire au dithiolane.
(ii) ou en deux étapes en formant un intermédiaire di-thiol, qui en présence d'un oxydant (oxygène, DMSO, FeCl₃, I₂, Br₂, iodure de sodium, trifluoroacetates de thallium, triflates d'argent, eau oxygénée, iodate et periodate de sodium, hypochlorite de sodium, ferricyanide de potassium, oxyde de chrome), en milieu neutre ou basique conduit à la formation du dithiolane. Dans ce cas, le di-thiol est obtenu par transformation (en milieu basique ou acide) dans un solvant polaire ou apolaire d'une espèce intermédiaire via des dérivés de l'acide thioacétique CH₃COSH (en présence de base), par de la thiourée ou du NaSH, par la formation de dithiosulfoantes (sels de Bunte).

La fonctionnalisation de l'acide carboxylique COOH en fonction COYR peut s'effectuer suivant les méthodes classiques d'activation des acides (décrites dans Comprehensive Organic Transformation de R. LAROCK Wiley VCH Ed. dans le chapitre Interconversion of nitriles, carboxylic acids and derivatives). De préférence les méthodes utilisées privilégient le passage au chlorure d'acide (par utilisation de chlorure de thionyle ou d'oxalyle, ou de 1-chloro-n.n.2-trimethyl-1-propenamine) ou par la formation d'anhydride mixte (à l'aide de chloroformiate d'alkyles) ou l'utilisation de carbodiimides ou de diéthylcyanophosphate (Phosphorus in organic synthesis-XI, Amino acids and peptides-XXI, Reaction of diethyl phosphorocyanidate with carboxylic acids. A new synthesis of carboxylic esters and amides, Tetrahedron, 32, 1976, 2211-2217). *

Les solvants utilisées peuvent être polaires ou apolaires, protiques ou aprotiques (par exemple toluène, dichlorométhane, THF, DMF, acétonitrile, eau, méthanol, isopropanol)

Toutes ces réactions peuvent s'effectuer à des températures comprises entre -20 et 100°C.

L'obtention des produits d'oxydation des atomes de soufre des dithiolanes de formule (I) (m et n différents de zéro) peut s'effectuer selon le schéma réactionnel suivant 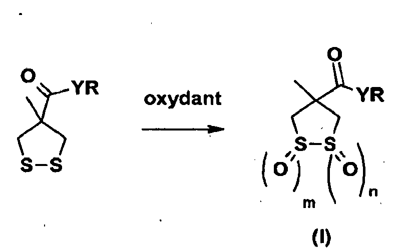 à l'aide d'oxydants tels que l'oxygène, l'eau oxygénée, le DMSO, le périodate de sodium, les peracides organiques, les persulfates inorganiques, les permanganates inorganiques en présence ou non de catalyseur (par exemple Na2WO4, MoO2Cl2, trichloro oxo bis triphenylphosphine rhenium). Les différentes étapes d'oxydation sont fonction de la stoechiométrie des oxydants utilisés. Les solvants utilisables peuvent être l'eau, l'acétone, le dichlorométhane, le méthanol.

Ces oxydations ont été décrites dans les documents suivants :
- Oxydation of 1,2-Dithiolanes, Bernt Lindberg, Göran Bergson, Arkiv För Kemi, 1965, 23(31), 319-333.
- Selective oxydation of sulfides to sulfoxides and sulfones at room temperature using H2O2 and a Mo(VI) salt as catalyst, Kandasamy Jeyakumar, Dillip Kumar Chand, Tetrahedrons Letters 47(2006), 4573-4576
- Rhenium-Catalyzed Oxidation of Thiols and Disulfides with Sulfoxides, Jeffrey B.Arterburn, Marc C.Perry, Sherry L.Nelson, Benjamin R.Dible, Mylena S. Holguin, J. Am. Soc, 119, 9309-9310, 1997

Avantageusement, le composé 1 peut être obtenu suivant la voie décrite ci-dessous à partir de l'acide dichloro-pivalique suivant un procédé en un pot, finalisé par un précipitation.

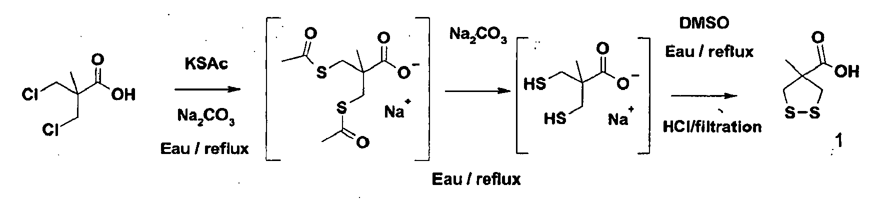

Avantageusement, le composé 2 peut être obtenu à partir du composé 1, préférentiellement à l'aide de chloroformiate d'isobutyle ou de chlorure d'oxallyle.

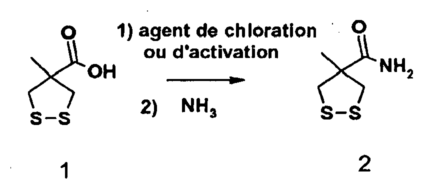

La Demanderesse a démontré que les UV journaliers (simulation de la lumière du jour UV moyenne, concept L'OREAL, Christiaens FJ and al, Standard ultraviolet daylight for non extreme exposure conditions, Photochem Photobiol, 2005) entraînait une diminution du taux de GSH intracellulaire sur des cellules HaCaT (kératinocytes issus d'un implant de peau humaine adulte spontanément immortalisé in vitro). La déplétion étant maximale 6 heures après exposition (diminution d'environ 40%) et retour à l'état basal 24 heures après l'exposition aux UV journaliers (UVJ).

Les composés selon l'invention peuvent prévenir et/ou corriger cette déplétion du GSH, ils peuvent ainsi « booster » les systèmes de défenses antioxydantes endogènes, afin de préparer la peau à mieux subir un stress UV et l'aider à se réparer. Leur activité a été comparée à un composé de référence, l'acide lipoïque, connu pour augmenter le taux de GSH.

La Demanderesse a ainsi montré que la capacité d'augmenter le taux de GSH des composés selon l'invention était bien supérieure à celle de l'acide lipoïque mesuré dans les mêmes conditions (voir l'exemple 17).

La présente invention a en particulier pour objet l'utilisation cosmétique d'au moins un composé dithiolane choisi parmi ceux de formule (I) dans une composition comprenant un milieu physiologiquement acceptable, dans le but de renforcer et/ou de préserver le taux de glutathion intracellulaire endogène apportant la protection antioxydante naturelle de la peau face au stress oxydatif notamment causé par les radiations UV.

Ainsi, l'utilisation selon l'invention permet de renforcer et/ou préserver les systèmes de défense antioxydante cellulaire, en particulier les systèmes de défense antioxydante des cellules de la peau. Les cellules de la peau sont notamment fibroblates, kératinocytes, cellules de Langerhans.

Selon une utilisation avantageuse de l'invention, les composés de formule générale (I) sont utiles comme agent photoprotecteur de la peau.

Cette utilisation peut être utile que la peau ait subi une exposition à la lumière du jour d'une intensité inférieure à la dose érythémale minimum et dont les effets ne produisent pas de signes visibles sur la peau ou bien que les dommages des rayons UV soient visibles, par exemple par l'apparition de rougeurs sur la peau.

En conséquence, les altérations vont du simple inconfort tel qu'une sensation d'échauffement de la peau uniquement perceptible à des rougeurs, voire des irritations.

Ainsi les composés de formule générale (I) sont utiles pour prévenir et/ou traiter les stress UV et/ou des sensations d'échauffement dus au rayonnement du soleil, en particulier UVA et /ou UVB.

Les composés de formule générale (I) sont aussi utiles pour la préparation d'une composition comprenant un milieu physiologiquement acceptable, destinée à la prévention et/ou au traitement d'altérations, telles que rougeurs et irritations cutanées, de la peau dues au rayonnement du soleil.

Les composés de formule générale (I) sont encore utiles pour la préparation d'une composition comprenant un milieu physiologiquement acceptable, destinée à la prévention et/ou au traitement des lésions à l'ADN dues au rayonnement du soleil et prévenir ainsi le développement de cancers, en particulier cutanés.

Les composés de formule générale (I) sont encore utiles pour la préparation d'une composition comprenant un milieu physiologiquement acceptable, destinée à la destinée au traitement des désordres de la peau et/ou des muqueuses induites par une irradiation par les rayonnements UVA et/ou UVB.

L'irradiation ou exposition solaire se caractérise par une exposition au soleil, il pourra notamment s'agir d'une irradiation intense correspondant à une exposition au soleil zénithal ou à des rayonnements solaires variant d'un angle de 30° autour de cette position zénithale et/ou lorsque la peau est soumise à un rayonnement UV capable d'induire un érythème solaire (rougeur appelée communément "coup de soleil"), et défini par une dose minimale érythémale (DME ou MED). Cette dose varie en fonction du phototype de l'individu et du rapport UVA/UVB.

L'invention vise notamment à prévenir ou diminuer les dommages induits dans la peau, les muqueuses et/ou les phanères d'un mammifère, en particulier d'un être humain, par des expositions courtes à des doses érythémateuses de rayonnement solaire.

Ces conditions d'exposition solaire comprennent des rayonnements UVA et/ou UVB, à des doses autour de la MED, en particulier à une dose supérieure ou égale à 1 MED.

Comme expliqué plus haut, de par leur capacité à augmenter le taux de GSH, les composés de formule (I) selon l'invention permettent la mise en place du système protecteur anti-oxydant cutané.

Aussi, l'utilisation cosmétique selon l'invention d'au moins un composé dithiolane choisi parmi ceux de formule (I) dans une composition renfermant un milieu physiologiquement acceptable est particulièrement bien adaptée pour préparer la peau à une exposition solaire.

En particulier, la préparation de la peau à une exposition solaire pourra être faite par l'application quotidienne sur la peau de ladite composition cosmétique pendant une semaine avant l'exposition solaire et, de préférence, pendant deux semaines, jusqu'à au moins une nuit (entre 6 et 18 heures) avant l'exposition solaire.

Qu'ils aient une origine endogène ou exogène, les radicaux libres provoquent des dégâts oxydatifs importants, notamment dans les membranes cellulaires (peroxydation de lipides provoquant une dégradation de la perméabilité des membranes), les noyaux des cellules (destruction de l'ADN), et les tissus, en particulier le tissu conjonctif (dégradation des fibres d'élastine et de collagène, dépolymérisation des fibres polyuroniques). Ces dégâts conduisent notamment à un dessèchement et à une perte de fermeté et d'élasticité de la peau (Grinwald et al. 1980, Agren et al. 1997).

Les spécialistes considèrent actuellement qu'une des causes du vieillissement cellulaire est l'amoindrissement des capacités de défense contre les radicaux libres et contre les phénomènes d'oxydation (notamment la formation d'ions superoxyde) qu'ils initient.

Ainsi, de façon plus générale, les composés de formule générale (I) selon l'invention sont utiles comme composés anti-oxydants indirects pour prévenir et/ou limiter la formation de radicaux libres et/ou éliminer les radicaux libres présents dans les cellules et peuvent être utilisés pour tout désordre cutané provoqué par un stress oxydatif.

Cette activité des composés de formule générale (I) est renforcée par la propriété antioxydante intrinsèque de ces composés liée à leur fonction thiol.

Ainsi l'utilisation des composés selon l'invention permet de prévenir et/ou traiter certains signes cliniques du vieillissement de la peau.

Le vieillissement en un phénomène physiologique naturel dont les signes cliniques peuvent se traduire généralement sur la peau par l'apparition de rides et ridules, par un relâchement des tissus cutanés et sous-cutanés, par une perte de l'élasticité cutanée et par une atonie de la texture de la peau. La perte de fermeté et de tonicité de la peau, comme les rides et les ridules, s'explique au moins en partie par une atrophie dermique et ainsi qu'un aplanissement de la jonction dermo-épidermique ; la peau est moins ferme et plus flasque, et l'épaisseur de l'épiderme diminue.

Un autre signe clinique du vieillissement est l'aspect sec et rêche de la peau qui est dû essentiellement à une desquamation plus importante ; ces squames, en diffractant les rayons lumineux, participent aussi l'aspect un peu gris du teint.

Certains de ces signes sont plus particulièrement liés au vieillissement intrinsèque ou physiologique, c'est-à-dire au vieillissement lié à l'âge, alors que d'autres sont plus spécifiques du vieillissement extrinsèque, c'est-à-dire du vieillissement provoqué d'une manière générale par l'environnement ; il s'agit plus particulièrement du photo-vieillissement dû l'exposition au soleil, la lumière ou tout autre rayonnement ou encore les polluants.

Ainsi l'objet de l'invention est particulièrement adapté à l'utilisation cosmétique d'au moins un composé de formule générale (I) selon l'invention dans une composition renfermant un milieu physiologiquement acceptable, pour prévenir et/ou traiter la perte de fermeté et/ou d'élasticité de la peau. Une telle utilisation permet notamment à la peau de retrouver un aspect uniformément lisse.

L'invention est également adaptée à l'utilisation cosmétique d'au moins un composé de formule générale (I) selon l'invention dans une composition renfermant un milieu physiologiquement acceptable, pour prévenir et/ou traiter la déshydratation cutanée.

De façon plus générale, l'objet de l'invention est également adapté à l'utilisation cosmétique d'au moins un composé choisi parmi ceux de formule générale (I) selon l'invention dans une composition renfermant un milieu physiologiquement acceptable, pour prévenir et/ou traiter l'atrophie épidermique et/ou la rugosité cutanée et/ou la sécheresse cutanée.

Un autre objet de l'invention se rapporte à l'utilisation cosmétique d'au moins un composé de formule générale (I) dans une composition renfermant un milieu cosmétiquement acceptable, pour prévenir et/ou traiter les effets néfastes de la pollution sur la peau.

On sait que la toxicité des polluants atmosphériques, notamment des polluants gazeux tels que le dioxyde de soufre, l'ozone et les oxydes d'azote sur les constituants de la peau (fibres, cellules, enzymes) et sur le sébum sécrété par la peau est liée notamment à leur activité d'initiateurs de radicaux libres, source de phénomènes d'oxydation qui provoquent chez les êtres vivants des dommages cellulaires.

Les cellules vivantes, qui sont en contact direct et permanent avec le milieu extérieur (notamment la peau, le cuir chevelu et certaines muqueuses) sont particulièrement sensibles à ces effets des polluants gazeux, qui se traduisent notamment par un vieillissement accéléré de la peau, avec une formation précoce de rides ou ridules, et aussi par une diminution de la vigueur et un aspect terne des cheveux.

Comme expliqué précédemment, un effet indésirable de la présence de radicaux libres dans la peau est qu'ils provoquent un phénomène de péroxydation des lipides. Avec l'âge (plus particulièrement à partir de quarante ans), l'accumulation de ces lipides péroxydés est responsable de mauvaises odeurs corporelles telles qu'une odeur rance (Haze S. et al. J. Invest. Dermatol. 2001, 116(4): 520-4).

L'objet de l'invention est adapté à l'utilisation cosmétique d'au moins un composé de formule générale (I) selon l'invention dans une composition renfermant un milieu physiologiquement acceptable, pour prévenir et/ou limiter et/ou éliminer la péroxydation des lipides cutanés.

Ainsi l'objet de l'invention est également utile pour prévenir et/ou limiter et/ou éliminer les mauvaises odeurs corporelles.

Les compositions selon l'invention pourront être à usage cosmétique et/ou dermatologique. Il peut s'agir de compositions adaptées à une application par voie topique, en particulier topique externe sur la peau, les muqueuses et/ou les phanères.

Selon un autre mode de réalisation, les compositions selon l'invention sont adaptées à la voie orale, et sont notamment destinées à des applications en cosmétique orale.

La quantité de composé utilisable selon l'invention est bien entendu fonction de l'effet recherché et peut donc varier dans une large mesure.

Pour donner un ordre de grandeur, on peut utiliser, pour la voie topique, en particulier topique externe, le ou les composés de formule (I), tels que définis précédemment en une quantité représentant de 0,001 % à 20% du poids total de la composition et préférentiellement en une quantité représentant de 0,01 % à 10% du poids total de la composition.

Pour la voie orale, on peut utiliser, le ou les composés de formule (I) tels que définis précédemment ledit composé est présent en une quantité comprise entre 0,1 et 100 mg par prise.

### Formulation des compositions

De préférence, les compositions selon l'invention comprenant au moins un composé de formule (I) est constituée d'un milieu cosmétiquement acceptable, c'est-à-dire qui présente une couleur, une odeur et un toucher agréable et qui ne génèrent pas d'inconfort inacceptable.

Les compositions sont de préférence des compositions ou produits cosmétique. Par "produit cosmétique", on entend notamment toute substance ou préparation destinée à être mise en contact avec les diverses parties superficielles du corps humain (épiderme, système pileux et capillaire, ongles, lèvres et organes génitaux externes) ou avec les dents et les muqueuses buccales en vue, exclusivement ou principalement, de les nettoyer, de les parfumer, d'en modifier l'aspect et/ou de corriger les odeurs corporelles et/ou de les protéger ou de les maintenir en bon état (directive cosmétique 76/768/CEE modifiée).

La composition selon l'invention peut notamment être sous forme de solution alcoolique, hydro-alcoolique ou huileuse, de suspension, de dispersion, d'émulsions E/H, H/E ou multiple, de gels aqueux ou anhydres, de dispersion vésiculaires de type ionique ou non ionique. Elle peut avoir une consistance liquide, semi-liquide, pâteuse, ou solide.

Pour une application topique, la composition utilisable selon l'invention peut être notamment sous la forme d'une solution hydroalcoolique ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H) ou multiples, d'une poudre libre ou compactée à utiliser telle quelle ou à incorporer dans un milieu physiologiquement acceptable, ou de suspensions ou émulsions de consistance molle du type crème ou gel aqueux ou anhydres, ou encore de microcapsules ou microparticules, ou de dispersions vésiculaires de type ionique et/ou non ionique. Elle peut ainsi se présenter sous forme d'onguent, de teinture, de crème, de pommade, de poudre, de timbre, de tampon imbibé, de solution, d'émulsion ou de dispersion vésiculaire, de lotion, de gel, de spray, de suspension, de shampooing, d'aérosol ou de mousse. Elle peut être anhydre ou aqueuse. Elle peut également consister en des préparations solides constituant des savons ou des pains de nettoyage.

Ces compositions sont préparées selon les méthodes usuelles.

Selon un autre mode de réalisation de l'invention, la composition est adaptée à un usage oral, en particulier en "cosmétique orale".

Pour un usage par voie orale, la composition peut notamment se présenter sous forme de capsules, gélules, dragées, de granulés, de comprimés, de pâte à mâcher, de gels ou de sirops buvables ou de toute autre forme connue de l'homme du métier.

Les quantités des différents constituants des compositions utilisables selon l'invention sont celles classiquement utilisées dans les domaines considérés.

### Associations

Les compositions selon l'invention peuvent contenir en outre des agents renforçant ou complétant l'activité du composé de formule (I), et notamment au moins composé choisi parmi les agents anti-oxydants, les agents anti-pollution, les filtres organiques et/ou les filtres minéraux, les agents stimulant la réparation de l'ADN.

Les composés de formule (I) selon l'invention pourront également être avantageusement associés à des extraits (biomasse totale, milieu de culture, fraction ribosomale, fraction de membranes cellulaires, fraction LPS, lipide A...) de bactéries filamenteuses non fructifiantes, non photosynthétiques telles que Vitreoscilla filiformis ou encore l'acide {2-[acetyl-(3-trifluoromethyl-phenyl)-amino]-3-methyl-butyrylamino}-acetique.

Ainsi, on peut par exemple utiliser un agent antioxydant choisi parmi :
- la vitamine E (tocophérol) et ses dérivés, entre autre acétate, linoléate ou nicotinate, de préférence à des concentrations de l'ordre de 0,1 à 5%,
- le γ-orizanol (0,1 à 5%),
- les pidolates de lysine ou d'arginine (0,5 à 10%),
- les extraits végétaux tels que l'extrait de mélisse (0,01 à 2%), l'extrait de silimarine (0,01 à 2%), l'extrait de gingko biloba (0,05 à 2%), l'extrait de sauge (0,05 à 2%), l'extrait de noix de cola (0,05 à 2%), l'extrait de rutine (0,1 à 2%) ou l'extrait de thym (0,1 à 2%), les % étant donnés en matière sèche,
- les caroténoides, tels que l'α et le β-carotène ou le lycopène sous une forme purifiée ou bien dans un extrait (par exemple pâte de tomate titrés en lycopène aboutissant à une concentration finale de lycopène comprise entre 10⁻¹²% et 10% et plus préférentiellement de 10⁻⁷% à 0,1%),
- les oligomères proanthocyanolidiques de pin, d'aubépine ou de raisin (0,1 à 2%),
- le di-tertiobutyl-hydroxybenzylidène camphre (0,1 à 2%),
- le thé vert (0,1 à 2%),
- la caféine (0,1 à 5%),
- le glycérol (2 à 30%),
- le mannitol (2 à 30%),
- la carnosine (0,1 à 2%),
- la superoxyde dismutase (100 à 10 000 UI/100 g),
- la guanosine (0,01 à 1 %),
- les microalgues contenant de l'ethoxyquine telles que l'Hématococcus (0,005 à 1%),
- le pentasodium aminotriméthylène phosphonate (0,001 à 0,5%),
- la lactoperoxydase (0,01 à 0,1%),
- la vitamine C et ses dérivés,
- la lactoferrine (0,01 à 0,1%),
- l'isopropyl (benzyl{2-[benzyl(2-isopropoxy-2-oxoethyl)amino]ethyl}amino)acetate,
- la phloretine,
- l'hesperidine,
- la neohesperidinedihydrochalcone,
- l'acide ferulique,
- les Eukarions (dont le EUK-8, EUK-134 et EUK-189 développés par Proteome Systems),
- l'acide L-2-oxo-4-thiazolidine carboxylique,
- l'ergothioneine,
- l'acide cafféique,
- le desferal,
- le 4,4'-(2,3-dimethylbutane-1,4-diyl)dibenzene-1,2-diol.
   On peut également utiliser un mélange de plusieurs agents antioxydants.

On peut également citer des agents anti-radicalaires, en particulier, les bioflavonoïdes ; le co-enzyme Q10 ou ubiquinone ; certaines enzymes comme la catalase, le glutathion peroxydase et les quinones réductases ; le glutathion ; le benzylidène camphre ; les benzylcyclanones ; les naphtalénones substituée ; les pidolates ; le phytantriol ; les lignanes ; la mélatonine ; les chalcones hydroxylées ainsi que leurs dérivés réduits.

De préférence, l'agent antioxydant est choisi parmi la vitamine C, la vitamine E, l'isopropyl (benzyl{2-[benzyl(2-isopropoxy-2-oxoethyl)amino]ethyl}amino)acetate, l'acide ferulique, la phlorétine, la néohespéridine dihydrochalcone et la SOD.
Par l'expression "agent anti-pollution", on entend tout composé capable de piéger l'ozone, les composés aromatiques mono- ou polycycliques tels que le benzopyrène et/ou les métaux lourds tels que le cobalt, le mercure, le cadmium et/ou le nickel. Par "agent anti-radicalaire", on entend tout composé capable de piéger les radicaux libres.

Comme agents piégeurs d'ozone utilisables dans la composition selon l'invention, on peut citer en particulier la vitamine C et ses dérivés dont le glucoside d'ascorbyle ; les phénols et polyphénols, en particulier les tannins, l'acide ellagique et l'acide tannique ; l'épigallocatéchine et les extraits naturels en contenant ; les extraits de feuille d'olivier ; les extraits de thé, en particulier de thé vert ; les anthocyanes ; les extraits de romarin ; les acides phénols, en particulier l'acide chorogénique ; les stilbènes, en particulier le resvératrol ; les dérivés d'acides aminés soufrés, en particulier la S-carboxyméthylcystéine ; l'ergothionéine ; la N-acétylcystéine ; des chélatants comme la N,N'-bis-(3,4,5-triméthoxybenzyl)éthylènediamine ou l'un de ses sels, complexes métalliques ou esters ; des caroténoïdes tels que la crocétine ; et des matières premières diverses comme le mélange d'arginine, ribonucléate d'histidine, mannitol, adénosinetriphosphate, pyridoxine, phénylalanine, tyrosine et ARN hydrolysé commercialisé par les Laboratoires Sérobiologiques sous la dénomination commerciale CPP LS 2633-12F^{®}, la fraction hydrosoluble de maïs commercialisée par la société SOLABIA sous la dénomination commerciale Phytovityl^{®}, le mélange d'extrait de fumeterre et d'extrait de citron commercialisé sous la dénomination Unicotrozon C-49^{®} par la société Induchem, et le mélange d'extraits de ginseng, de pomme, de pêche, de blé et d'orge vendu par la société PROVITAL sous la dénomination commerciale Pronalen Bioprotect^{®}.

Comme agents piégeurs de composés aromatiques mono- ou polycycliques utilisables dans la composition selon l'invention, on peut citer en particulier les tannins tels que l'acide ellagique ; les dérivés indoles, en particulier l'indol-3-carbinol ; les extraits de thé en particulier de thé vert, les extraits de Jacinthe d'eau ou eichornia crassipes ; et la fraction hydrosoluble de maïs commercialisée par la société SOLABIA sous la dénomination commerciale Phytovityl^{®}.

Enfin, comme agents piégeurs de métaux lourds utilisables dans la composition selon l'invention, on peut citer en particulier les agents chélatants tels que l'EDTA, le sel pentasodique d'éthylènediamine tétraméthylène phosphonique, et la N,N'-bis-(3,4,5-triméthoxybenzyl)éthylènediamine ou l'un de ses sels, complexes métalliques ou esters ; l'acide phytique ; les dérivés de chitosan ; les extraits de thé, en particulier de thé vert ; les tannins tels que l'acide ellagique ; les acides aminés soufrés tels que la cystéine ; les extraits de Jacinthe d'eau (Eichornia crassipes) ; et la fraction hydrosoluble de maïs commercialisée par la société SOLABIA sous la dénomination commerciale Phytovityl^{®}.

Avantageusement, les compositions selon l'invention comportent en outre au moins un agent photoprotecteur organique et/ou au moins un agent photoprotecteur inorganique actif dans l'UVA et/ou l'UVB (absorbeurs), hydrosolubles ou liposolubles ou bien insolubles dans les solvants cosmétiques couramment utilisés. De préférence, on utilisera un système filtrant à la fois les radiations UVA et les radiations UVB.

Les filtres solaires sont des molécules qui absorbent les rayonnements UV et permettent ainsi d'éviter que ceux-ci n'arrivent au niveau des cellules de la peau. Ils peuvent absorber soit principalement les UVB, soit principalement les UVA, en fonction de leur nature. Il existe deux grandes catégories de filtres solaires, soit organiques, soit minéraux (oxydes de zinc ou de titane). En les utilisant dans des compositions cosmétiques en association et en quantité suffisante, ils permettent d'arrêter une grande partie du rayonnement UV.

Cependant il est couramment admis que pour être efficaces ces formules doivent être utilisées dans de bonnes conditions d'application (quantité suffisante, renouvellement fréquent, étalement homogène). Ces conditions d'application ne sont pas toujours respectées par l'utilisateur, ce qui augmente le risque qu'une quantité non négligeable de rayonnements UV atteigne les cellules de la peau, et donc engendre les effets biologiques cités plus haut. De plus, pour obtenir une absorption vis-à-vis de l'ensemble des longueurs d'onde du spectre UV solaire UVB + UVA il faut associer plusieurs molécules absorbant dans des domaines de longueurs d'onde complémentaires.

Les filtres organiques complémentaires sont notamment choisis parmi les anthranilates ; les dérivés cinnamiques ; les dérivés salicyliques, les dérivés du camphre ; les dérivés de la benzophénone ; les dérivés de β ,β-diphénylacrylate ; les dérivés de triazine ; les dérivés de benzotriazole ; les dérivés de benzalmalonate notamment ceux cités dans le brevet US5624663 ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US 5,166,355, GB2303549, DE 197 26 184 et EP893119 ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 ; les dérivés de mérocyanines tels que ceux décrits dans les demandes WO04006878, WO05058269 et WO06032741 et leurs mélanges .

Comme exemples d'agents photoprotecteurs organiques complémentaires, on peut citer ceux désignés ci-dessous sous leur nom INCI :

### Dérivés cinnamiques :

Ethylhexyl Methoxycinnamate vendu notamment sous le nom commercial PARSOL MCX par DSM Nutritional Products, Inc"
Isopropyl Methoxycinnamate
Isoamyl Methoxycinnamate vendu sous le nom commercial NEO HELIOPAN E 1000 par SYMRISE,
DEA Methoxycinnamate,
Diisopropyl Methylcinnamate,
Glyceryl Ethylhexanoate Dimethoxycinnamate

Dérivés de l'acide para-aminobenzoique :
PABA,
Ethyl PABA,
Ethyl Dihydroxypropyl PABA,
Ethylhexyl Diméthyl PABA vendu notamment sous le nom « ESCALOL 507 » par ISP,
Glyceryl PABA,
PEG-25 PABA vendu sous le nom « UVINUL P25 » par BASF,

### Dérivés salicyliques :

Homosalate vendu sous le nom « Eusolex HMS » par Rona/EM Industries,
Ethylhexyl Salicylate vendu sous le nom « NEO HELIOPAN OS » par SYMRISE,
Dipropyleneglycol Salicylate vendu sous le nom « DIPSAL » par SCHER,
TEA Salicylate, vendu sous le nom « NEO HELIOPAN TS » par SYMRISE,

### Dérivés de β ,β-diphénylacrylate :

Octocrylene vendu notamment sous le nom commercial « UVINUL N539 » par BASF,
Etocrylene, vendu notamment sous le nom commercial « UVINUL N35 » par BASF,

### Dérivés de la benzophénone :

Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-4 vendu sous le nom commercial « UVINUL MS40 » par BASF,
Benzophenone-5
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial« UVINUL DS-49» par BASF,
Benzophenone-12
2-(4-diethylamino-2-hydroxybenzoyl)-benzoate de n-hexyle vendu sous le nom commercial « UVINUL A+ » par BASF.

### Dérivés du benzylidène camphre :

3-Benzylidene camphor fabriqué sous le nom « MEXORYL SD» par CHIMEX,
4-Methylbenzylidene camphor vendu sous le nom « EUSOLEX 6300 » par MERCK , Benzylidene Camphor Sulfonic Acid fabriqué sous le nom « MEXORYL SL» par CHIMEX,
Camphor Benzalkonium Methosulfate fabriqué sous le nom « MEXORYL SO » par CHIMEX,
Terephthalylidene Dicamphor Sulfonic Acid fabriqué sous le nom « MEXORYL SX » par CHIMEX,
Polyacrylamidomethyl Benzylidene Camphor fabriqué sous le nom « MEXORYL SW » par CHIMEX,

### Dérivés du phenyl benzimidazole :

Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial « NEO HELIOPAN AP » par SYMRISE,

### Dérivés du phenyl benzotriazole :

Drometrizole Trisiloxane vendu sous le nom « Silatrizole » par RHODIA CHIMIE , Methylène bis-Benzotriazolyl Tetramethylbutylphénol, vendu sous forme solide sous le nom commercial « MIXXIM BB/100 » par FAIRMOUNT CHEMICAL ou sous forme micronisée en dispersion aqueuse sous le nom commercial « TINOSORB M » par CIBA SPECIALTY CHEMICALS,

### Dérivés de triazine :

- Bis-Ethylhexyloxyphenol Methoxyphenyl Triazine vendu sous le nom commercial «TINOSORB S » par CIBA GEIGY,
- Ethylhexyl triazone vendu notamment sous le nom commercial «UVINUL T150 » par BASF,
- Diethylhexyl Butamido Triazone vendu sous le nom commercial « UVASORB HEB » par SIGMA 3V,
- la 2,4,6-tris(4'-amino benzalmalonate de dinéopentyle)-s-triazine
- la 2,4,6-tris-(4'-amino benzalmalonate de diisobutyle)-s- triazine,
- la 2,4bis (4'-aminobenzoate de n-butyle)-6-(aminopropyltrisiloxane)-s-triazine,
- la 2,4bis (4'-aminobenzalmalonate de dinéopentyle)-6-(4'-aminobenzoate de n-butyle)-s-triazine,
- les filtres triazines symétriques décrits dans le brevet US6,225,467, la demande WO2004/085412 (voir composés 6 et 9) ou le document « Symetrical Triazine Derivatives » IP.COM Journal , IP.COM INC WEST HENRIETTA, NY, US (20 septembre 2004) notamment les 2,4,6-tris-(biphényl)-1,3,5-triazines (en particulier la 2,4,6-tris(biphenyl-4-yl-1,3,5-triazine et la 2,4,6-tris(terphenyl)-1,3,5-triazine qui est repris dans les demandes de brevet WO06/035000, WO06/034982, WO06/034991, WO06/035007, WO2006/034992, WO2006/034985.

### Dérivés anthraniliques :

Menthyl anthranilate vendu sous le nom commercial commercial « NEO HELIOPAN MA » par Haarmann et REIMER,

### Dérivés d'imidazolines :

Ethylhexyl Dimethoxybenzylidene Dioxoimidazoline Propionate,

### Dérivés du benzalmalonate :

Polyorganosiloxane à fonctions benzalmalonate comme le Polysilicone-15 vendu sous la dénomination commerciale « PARSOL SLX » par DSM Nutritional Products, Inc

### Dérivés de 4,4-diarylbutadiène :

- 1,1-dicarboxy (2,2'-diméthyl-propyl)-4,4-diphénylbutadiène

### Dérivés de benzoxazole :

2,4-bis-[5-1 (diméthylpropyl)benzoxazol-2-yl-(4-phenyl)-imino]-6-(2-ethylhexyl)-imino-1,3,5-triazine vendu sous le nom d'Uvasorb K2A par Sigma 3V et leurs mélanges.

### Dérivés de mérocyanine

Octyl-5-N,N-diethylamino-2-phenysulfonyl-2,4-pentadienoate

Les filtres organiques préférentiels sont choisis parmi
Ethylhexyl Methoxycinnamate
Ethylhexyl Salicylate,
Homosalate,
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
Methylène bis-Benzotriazolyl Tetramethylbutylphénol
Bis-Ethylhexyloxyphenol Methoxyphenyl Triazine
Ethylhexyl triazone,
Diethylhexyl Butamido Triazone,
la 2,4,6-tris(4'-amino benzalmalonate de dinéopentyle)-s-triazine
la 2,4,6-tris-(4'-amino benzalmalonate de diisobutyle)-s- triazine
la 2,4bis (4'-aminobenzoate de n-butyle)-6-(aminopropyltrisiloxane)-s-triazine,
la 2,4bis (4'-aminobenzalmalonate de dinéopentyle)-6-(4'-aminobenzoate de n-butyle)-s-triazine,
la 2,4,6-tris(biphenyl-4-yl-1,3,5-triazine
la 2,4,6-tris(terphenyl)-1,3,5-triazine
Drometrizole Trisiloxane
Polysilicone-15
1,1-dicarboxy (2,2'-diméthyl-propyl)-4,4-diphénylbutadiène
2,4-bis-[5-1(diméthylpropyl)benzoxazol-2-yl-(4-phenyl)-imino]-6-(2-ethylhexyl)-imino-1,3,5-triazine
Octyl-5-N,N-diethylamino-2-phenysulfonyl-2,4-pentadienoate et leurs mélanges.

Les filtres organiques conformes à l'invention représentent en général de 0,1 à 30 %, de préférence de 1 à 25 %, du poids total de la composition.

Les filtres UV inorganiques complémentaires utilisés conformément à la présente invention sont des pigments d'oxyde métallique. Plus préférentiellement, les filtres UV inorganiques de l'invention sont des particules d'oxyde métallique ayant une taille moyenne de particule élémentaire inférieure ou égale à 500 nm, plus préférentiellement comprise entre 5 nm et 500 nm, et encore plus préférentiellement comprise entre 10 nm et 100 nm, et préférentiellement entre 15 et 50 nm.

Ils peuvent être notamment choisis parmi les oxydes de titane, de zinc, de fer, de zirconium, de cérium ou leurs mélanges et plus particulièrement les oxydes de titane.

De tels pigments d'oxydes métalliques, enrobés ou non enrobés sont en particulier décrits dans la demande de brevet EP-A- 0 518 773. A titre de pigments commerciaux on peut mentionner les produits vendus les sociétés Kemira, Tayca, Merck et Degussa.

Les pigments d'oxydes métalliques peuvent être enrobés ou non enrobés.

Les pigments enrobés sont des pigments qui ont subi un ou plusieurs traitements de surface de nature chimique, électronique, mécanochimique et/ou mécanique avec des composés tels que des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

Les pigments enrobés sont plus particulièrement des oxydes de titane enrobés :
- de silice tels que le produit "SUNVEIL" de la société IKEDA,
- de silice et d'oxyde de fer tels que le produit "SUNVEIL F" de la société IKEDA,
- de silice et d'alumine tels que les produits "MICROTITANIUM DIOXIDE MT 500 SA" et"MICROTITANIUM DIOXIDE MT 100 SA" de la société TAYCA, "TIOVEIL" de la société TIOXIDE,
- d'alumine tels que les produits "TIPAQUE TTO-55 (B)" et "TIPAQUE TTO-55 (A)" de la société ISHIHARA, et "UVT 14/4" de la société KEMIRA,
- d'alumine et de stéarate d'aluminium tels que les produits "MICROTITANIUM DIOXIDE MT 100 T, MT 100 TX, MT 100 Z, MT-01" de la société TAYCA, les produits "Solaveil CT-10 W" et "Solaveil CT 100" de la société UNIQEMA et le produit " Eusolex T-AVO" de la société MERCK,
- de silice, d'alumine et d'acide alginique tel que le produit " MT-100 AQ" de la société TAYCA,
- d'alumine et de laurate d'aluminium tel que le produit "MICROTITANIUM DIOXIDE MT 100 S" de la société TAYCA,
- d'oxyde de fer et de stéarate de fer tels que le produit "MICROTITANIUM DIOXIDE MT 100 F" de la société TAYCA,
- d'oxyde de zinc et de stéarate de zinc tels que le produit "BR 351" de la société TAYCA,
- de silice et d'alumine et traités par une silicone tels que les produits "MICROTITANIUM DIOXIDE MT 600 SAS", "MICROTITANIUM DIOXIDE MT 500 SAS" ou "MICROTITANIUM DIOXIDE MT 100 SAS"de la société TAYCA,
- de silice, d'alumine, de stéarate d'aluminium et traités par une silicone tels que le produit "STT-30-DS" de la société TITAN KOGYO,
- de silice et traité par une silicone tel que le produit "UV-TITAN X 195" de la société KEMIRA,
- d'alumine et traités par une silicone tels que les produits "TIPAQUE TTO-55 (S)" de la société ISHIHARA, ou "UV TITAN M 262" de la société KEMIRA,
- de triéthanolamine tels que le produit "STT-65-S" de la société TITAN KOGYO,
- d'acide stéarique tels que le produit "TIPAQUE TTO-55 (C)" de la société ISHIHARA,
   d'hexamétaphosphate de sodium tels que le produit "MICROTITANIUM DIOXIDE MT 150 W" de la société TAYCA.
- le TiO₂ traité par l'octyl triméthyl silane vendu sous la dénomination commerciale "T 805" par la société DEGUSSA SILICES,
- le TiO₂ traité par un polydiméthylsiloxane vendu sous la dénomination commerciale "70250 Cardre UF TiO2SI3" par la société CARDRE,
- le TiO₂ anatase/rutile traité par un polydiméthylhydrogénosiloxane vendu sous la dénomination commerciale "MICRO TITANIUM DIOXYDE USP GRADE HYDROPHOBIC" par la société COLOR TECHNIQUES.

Les pigments d'oxyde de titane non enrobés sont par exemple vendus par la société TAYCA sous les dénominations commerciales "MICROTITANIUM DIOXIDE MT 500 B" ou "MICROTITANIUM DIOXIDE MT600 B", par la société DEGUSSA sous la dénomination "P 25", par la société WACKHER sous la dénomination "Oxyde de titane transparent PW", par la société MIYOSHI KASEI sous la dénomination "UFTR", par la société TOMEN sous la dénomination "ITS" et par la société TIOXIDE sous la dénomination "TIOVEIL AQ".

Les pigments d'oxyde de zinc non enrobés, sont par exemple :
- ceux commercialisés sous la dénomination "Z-cote" par la société Sunsmart ;
- ceux commercialisés sous la dénomination "Nanox" par la société Elementis ;
- ceux commercialisés sous la dénomination "Nanogard WCD 2025" par la société Nanophase Technologies ;

Les pigments d'oxyde de zinc enrobés sont par exemple :
- ceux commercialisés sous la dénomination "Oxide zinc CS-5" par la société Toshibi (ZnO enrobé par polymethylhydrogenesiloxane) ;
- ceux commercialisés sous la dénomination "Nanogard Zinc Oxide FN" par la société Nanophase Technologies (en dispersion à 40% dans le Finsolv TN, benzoate d'alcools en C₁₂-C₁₅) ;
- ceux commercialisés sous la dénomination "DAITOPERSION ZN-30" et "DAITOPERSION Zn-50" par la société Daito (dispersions dans cyclopolyméthylsiloxane /polydiméthylsiloxane oxyéthyléné, contenant 30% ou 50% de nano-oxydes de zinc enrobés par la silice et le polyméthylhydrogènesiloxane) ;
- ceux commercialisés sous la dénomination "NFD Ultrafine ZnO" par la société Daikin (ZnO enrobé par phosphate de perfluoroalkyle et copolymère à base de perfluoroalkyléthyle en dispersion dans du cyclopentasiloxane) ;
- ceux commercialisés sous la dénomination "SPD-Z1" par la société Shin-Etsu (ZnO enrobé par polymère acrylique greffé silicone, dispersé dans cyclodiméthylsiloxane) ;
   ceux commercialisés sous la dénomination "Escalol Z100" par la société ISP (ZnO traité alumine et dispersé dans le mélange methoxycinnamate d'ethylhexyle / copolymère PVP-hexadecene / methicone) ;
- ceux commercialisés sous la dénomination "Fuji ZnO-SMS-10" par la société Fuji Pigment (ZnO enrobé silice et polymethylsilsesquioxane) ;
- ceux commercialisés sous la dénomination "Nanox Gel TN" par la société Elementis (ZnO dispersé à 55% dans du benzoate d'alcools en C₁₂-C₁₅ avec polycondensat d'acide hydroxystéarique).

Les pigments d'oxyde de cérium non enrobés peuvent être par exemple ceux vendus sous la dénomination "COLLOIDAL CERIUM OXIDE" par la société RHONE POULENC.

Les pigments d'oxyde de fer non enrobés sont par exemple vendus par la société ARNAUD sous les dénominations "NANOGARD WCD 2002 (FE 45B)", "NANOGARD IRON FE 45 BL AQ", "NANOGARD FE 45R AQ, "NANOGARD WCD 2006 (FE 45R)", ou par la société MITSUBISHI sous la dénomination "TY-220".

Les pigments d'oxyde de fer enrobés sont par exemple vendus par la société ARNAUD sous les dénominations "NANOGARD WCD 2008 (FE 45B FN)", "NANOGARD WCD 2009 (FE 45B 556)", "NANOGARD FE 45 BL 345", "NANOGARD FE 45 BL", ou par la société BASF sous la dénomination "OXYDE DE FER TRANSPARENT".

On peut également citer les mélanges d'oxydes métalliques, notamment de dioxyde de titane et de dioxyde de cérium, dont le mélange équipondéral de dioxyde de titane et de dioxyde de cérium enrobés de silice, vendu par la société IKEDA sous la dénomination "SUNVEIL A", ainsi que le mélange de dioxyde de titane et de dioxyde de zinc enrobé d'alumine, de silice et de silicone tel que le produit "M 261" vendu par la société KEMIRA ou enrobé d'alumine, de silice et de glycérine tel que le produit "M 211" vendu par la société KEMIRA.

Selon l'invention, les pigments d'oxyde de titane, enrobés ou non enrobés, sont particulièrement préférés.

Les filtres inorganiques conformes à l'invention représentent en général de 0,5 à 40 %, de préférence de 1 à 30 %, du poids total de la composition.

Les filtres inorganiques peuvent être introduits dans les compositions selon l'invention tels quels ou sous forme de pâte pigmentaire, c'est-à-dire en mélange avec un dispersant, comme décrit par exemple dans le document GB-A-2206339.

Un dérivé de vitamine E utilisable est notamment l'acétate de tocophéryle.

Les agents stimulant la réparation de l'ADN sont notamment des enzymes favorisant sa réparation telles que la photolyase et/ou l'endonucléase T4.

La composition peut en outre contenir des agents hydratants, des inhibiteurs de NO-synthase, des agents anti-radicalaires ou des agents stimulant la synthèse de macromolécules épidermiques et/ou empêchant leur dégradation.

Les quantités de ces différents composants seront adaptées par l'homme du métier.

Les exemples qui suivent servent à illustrer l'invention. Dans les exemples de formulation, les quantités des ingrédients compositions sont données en % pondéral par rapport au poids total de la composition.

### Exemples de synthèse:

### Exemple 1 : synthèse de l'acide 4-methyl-1,2-dithiolane-4-carboxylique (composé 1)

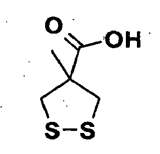

8g d'acide dichloropivalique sont placés dans un tricol de 250ml surmonté d'un réfrigérant et d'une ampoule de coulée. L'acide est dissout dans 80ml d'eau, en ajoutant lentement 4,6g de Na₂CO₃. Une solution de 10,7 g de thioacétate de potassium est additionnée goutte à goutte, le milieu réactionnel est porté au reflux. 14,9 g de Na₂CO₃ sont ajoutés et le milieu chauffé de nouveau au reflux. Après disparition du produit de départ, 7,3 ml de DMSO sont ajoutés avant de chauffer au reflux. Le dithiolane est obtenu après acidification par précipitation et séchage sous vide du solide. On obtient un solide jaune pâle
RMN 1 H (400 MHz, DMSO-d6): δ ppm 3,69 (d, 2H), 2,95 (d, 2H), 1,53 (s, 3H), ESI- :
[(M, H) -] = 163 m/z

### Exemple 2: Synthèse de l'octyl 4-methyl-1,2-dithiolane-4-carboxylate (composé 9)

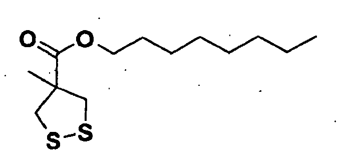

Dans un tricol de 100 ml, sont chargés sous argon 1g d'acide (24) puis 0,8 ml du 1-chloro-N,N-2-triméthyl-propenylamine (27) à l'aide d'une seringue dans 20 ml de dichlorométhane. le mélange est agité pendant 1 h puis introduit goutte à goutte par une ampoule de coulée dans un milieu réactionnel à -5°C contenant 1.28ml de triéthylamine, 0,96 ml d'octanol et 20 ml de dichlorométhane. On laisse agiter. Le milieu réactionnel est ensuite lavé à l'eau (3 X30 ml). La phase aqueuse est extraite avec 3X10ml d'AcOEt . Les phases organiques réunies sont lavées avec 30ml de solution aqueuse saturée de NaCl puis séchées sur Na₂SO₄, filtrées puis concentrées sous vide (500mbar, T=40°C) au rotavapor. Le brut obtenu est une huile jaune (m=1,25g). La purification se fait par chromatographie flash sur une colonne de silice (mSiO₂=40g- Eluant par gradient Heptane/AcOEt : 100/0 puis 98/2.)

Après concentration des fractions au rotavapor (P=100 mbar, T=40°C), on obtient 1,08 g d'attendu pur.
Huile jaune Rendement =66% ; Rf (ester)= 0,16 (Eluant : Cyclohexane) ;
RMN 1 H (400 MHz, DMSO-d6): δ ppm 4,08 (t, 2H), 3,57 (d, 2H), 3,02 (d, 2H), 1,58 (m, 2H), 1,40 (s, 3H), 1.29 (m, 10H), 0,86 (t, 3H)
MS m/z (M+, 277 ; M+23, 299).

Les manipulations suivantes ont été faites dans les mêmes conditions précédemment décrites, seul le nucléophile varie.

### Exemple 3 : Synthèse du S-[2-(acetylamino)ethyl] 4-methyl-1,2-dithiolane-4-carbothioate (Composé 13)

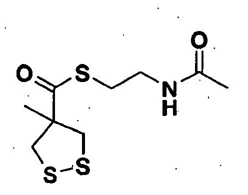

Méthode identique à l'exemple 2 : le nucléophile utilisé est la N-acétylcystéamine (0,64ml).

La purification se fait par chromatographie flash sur une colonne de silice (mSiO2=40g- Eluant par gradient linéaire DCM/MeOH : 100/0 puis 98/2.)

Après concentration des fractions au rotavapor (P=200 mbar, T=40°C), on obtient 0,32 g d'un mélange de l'attendu avec le N,N-2-triméthyl-propionamide (28).
Liquide jaune épaisse Rendement=10% ; Rf (attendu)= 0,3 ; Eluant : DCM/MeOH : 95/5 ; RMN 1 H (DMSO-d6): δ ppm 8,03 (t, NH), 3,57 (d, 2H), 3,18 (d.t 2H), 3,10 (d, 2H), 2,.96 (m, 2H), 1,79 (s, 3H), 1,43 (s, 3H) ; MS m/z (M+, 266 ; M+23, 288).

### Exemple 4 : Synthèse du N-(2-hydroxyethyl)-4-methyl-1,2-dithiolane-4-carboxamide (Composé 14)

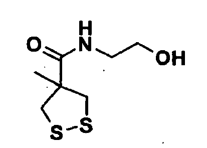

Méthode identique à l'exemple 2 : le nucléophile utilisé est l'éthanolamine (0,36ml). Après filtration du milieu réactionnel, on obtient un brut, huile jaune (m=1,850g)

La purification se fait par chromatographie flash sur une colonne de silice (Eluant par gradient linéaire DCM/MeOH : 100/0 puis 98/2.

Après concentration des fractions au rotavapor (P=500 mbar, T=40°C), on obtient 800 mg d'attendu pur, huile jaune R=65%
Rf (attendu)= 0,43 ; Eluant : DCM/MeOH : 9/1 ;
RMN 1 H (DMSO-d6): δ ppm 7,80 (t, NH), 4,64 (t, OH), 3,53 (d. 2H), 3,40 (d.t, 2H), 3,14 (m, 2H), 2,99 (d, 2H), 1,34 (s, 3H) ; MS m/z (M+, 208 ; M+23, 230).

### Exemple 5 : Synthèse du 4-methyl-1,2-dithiolane-4-carboxamide (Composé 2)

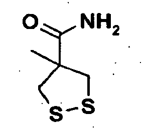

Méthode (ex5-a) identique à l'exemple 2 : le nucléophile utilisé est l'ammoniaque dans l'isopropanol (9,5ml). Après filtration du milieu réactionnel, on obtient un brut, huile jaune (m=1.853g).

La purification se fait par chromatographie flash sur une colonne de silice (Eluant : DCM).

Après concentration des fractions au rotavapor (P=600 mbar, T=40°C), on obtient 500 mg d'attendu pur, solide jaune Rendement =52%.

Alternativement, méthode (ex5-b), à une solution de 1g de composé 1 dans le THF avec 1,2 équivalent de triéthylamine, sont ajoutés à 0°C 1,2 équivalent de chloroformiate d'isobutyle. Après 2 heures à température ambiante, le milieu réactionnel est ajouté à de l'ammoniac en solution refroidie, soit dans l'eau à 28% soit 2N dans l'isopropanol. Le milieu est agité à température ambiante le temps nécessaire avant concentration sous vide. Le brut est alors repris dans le toluène pour conduire par précipitation au composé 2. Rendement = 60%

Rf (attendu)= 0,45 ; Eluant : DCM/MeOH : 95/5 ; RMN 1 H (DMSO-d6): δ ppm 7,38 (s, NH), 7,13 (s, NH), 3,53 (d. 2H), 2,97 (d, 2H), 1,34 (s, 3H) ; ESI- : [(M, H) -] = 162 m/z ; ESI+ : [(M, Na) +] = 186 m/z ; ESI+ : [(M, H) +] = 164 m/z ; ESI+ : [(M, Na, MeOH) +] = 218 m/z

### Exemple 6 : Synthèse du N-(2,3-dihvdroxypropyl)-4-methyl-1,2-dithiolane-4-carboxamide (Composé 15)

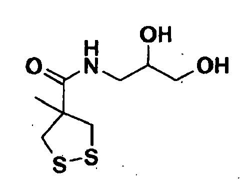

Méthode identique à l'exemple 2 : la quantité d'acide de départ engagé est 0,.25 g et le nucléophile utilisé est le diméthyldioxalaneméthanamine (0.2ml).

On obtient 110mg d'attendu pur, huile jaune Rendement=26%
Rf (attendu)= 0,51 ; Eluant : DCM/MeOH : 95/5 ; RMN 1 H (DMSO-d6): δ ppm 7.77 (t, 1 H :NH), 3,55 (d.d 4H, H3 :diastéréoisomères), 3,5 (m, 4H : H7 diastéréoisomères), 3,20 (m, 2H : H8 : diastéréoisomères), 3,05 (d.d, 2H:H9 et H9'), 2,99 (d.d, 4H, H5), 1,35 (s, 12H, H10+H11: diastéréoisomères), 0,9 (d, 3H, H6); MS m/z (M+23, 300).
70mg du produit pur protégé sous forme d'acétonide et environ 5 g de résine Dowex sont engagés dans une solution de 3ml d'eau et 2ml de THF. Le mélange réactionnel est agité à TA pendant 20h, puis à 40°C pendant 40h.
Le milieu réactionnel avec de la résine est filtré sous vide et lavé avec 3*10 ml d'eau puis 2X10ml d'EtOH. Le filtrat est ensuite concentré au rotavapor (P=200 mbar, T=40°C), on obtient 30 mg d'huile jaune présentant 2 diastéréoisomères.
Rf (attendu)= 0,24 ; Eluant : DCM/MeOH : 9/1 ; RMN 1 H (DMSO-d6): δ ppm 7,80 (t, 1 H : NH), 4.73 (d, OH), 4.50 (t, OH), 3,55 (d. 4H), 3,4 (m. 2H), 3.,2 (m, 1 H), 3.1 (m, 2H), 2,99 (d, 4H), 1,35 (s, 3H) ; MS m/z (M+, 208 ; M+23, 230).

### Exemple 7 : Synthèse du N-heptyl-4-methyl-1,2-dithiolane-4-carboxamide (Composé 10)

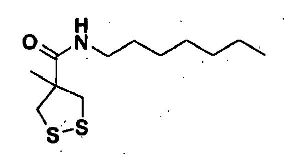

Méthode identique à l'exemple 2 : le nucléophile est 0.22ml de n-Heptylamine.
Le brut obtenu est une huile jaunâtre (m=0,27g).
La purification se fait par chromatographie flash sur une colonne de silice (mSiO₂=12g Eluant DCM/MeOH : 99/1)
Après concentration des fractions au rotavapor (P=500 mbar, T=40°C), on obtient 0.21 g d'attendu pur, huile jaune Rendement=54%.
Rf (attendu)= 0.5 ; Eluant : DCM/MeOH : 99/1 ; RMN 1 H (DMSO-d6): δ ppm 7.78 (t,
NH), 3,53 (d. 2H), 3.1 (d.t, 2H), 2,97 (d, 2H), 1,41 (t.t. 2H), 1,34 (s, 3H), 1,23 (m, 8H), 0,85 (t, 3H) ; MS m/z (M+, 262 ; M+23, 284)

### Exemple 8 : Synthèse du Methyl 2-{[(4-methyl-1,2-dithiolan-4-yl)carbonyl]amino}-4-(methylsulfanyl)butanoate (Composé 12)

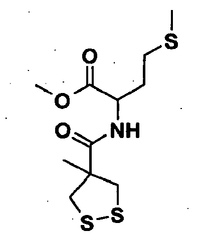

Méthode identique à l'exemple 2 : le nucléophile est la L-méthionine methyl-ester. RMN 1 H (DMSO-d6): δ ppm 8,13 (d, NH), 4,4 (m. 1 H), 3.63 (s, 3H), 3,58 (m, 2H), 3,02 (m, 2H), 2,5 (m, 2H), 2.04 (s, 3H), 1,96 (m, 2H), 1,38 (s, 3H); MS m/z (M+, 310 ; M+23, 332)

### Exemple 9 : Synthèse du N-butyl-4-methyl-1,2-dithiolane-4-carboxamide (Composé 11)

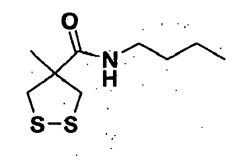

A 1g d'acide 1,2 dithiolane 4-méthyl 4-carboxylique dans ml de THF anhydre sont ajoutés 1,1 équivalent de triéthylamine et 1, équivalent de diéthylcyanophosphate à 0°C. 1,1 équivalent de n-butylamine sont ajoutés à 0°C et le milieu est agité pendant 1 heure avec un retour à température ambiante. Après évaporation et traitement aqueux par extraction, le brut réactionnel reconcentré est purifié sur colonne de silice (éluant dichlorométhane). Après évaporation des fractions d'intérêt on obtient une huile jaune.
RMN 1 H (DMSO-d6): δ ppm 7,79 (t, NH), 3,53 (d. 2H), 3,1 (d.t, 2H), 2,97 (d, 2H), 1,41 (t.t. 2H), 1,34 (s, 3H), 1,23 (m, 8H), 0,85 (t, 3H) ; MS m/z (M+, 262 ; M+23, 284)
RMN 1 H (DMSO-d6): δ ppm 7,79 (t, NH), 3,54 (d. 2H), 3,08 (d.t, 2H), 2,98 (d, 2H), 1,40 (q 2H), 1,34 (s, 3H), 1,27 (m, 4H), 0,87 (t, 3H) ; ESI+ : [(M, Na) +] = 242 m/z

### Exemple 10 : Synthèse du 4-methyl-1,2-dithiolane-4-carboxylic acid 1-oxide (Composé 23)

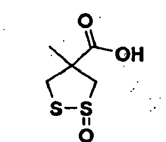

A 100mg d'acide 4-methyl-1,2-dithiolane-4-carboxylique dans 2 ml d'acétone, sont ajoutés 1 équivalent d'eau oxygénée à 30%. Le milieu réactionnel est agité à 20°C pendant une nuit. Après concentration sous vide, on obtient quantitativement le thiosulfinate sous forme d'un solide blanc mélange de 2 diastéréoisomères en proportion 70/30.
RMN 1 H (DMSO-d6): δ ppm
Diastéréoisomère majoritaire : 4,38 (d, 1 H), 3,78 (q, 2H), 3,11 (d, 1 H), 1,57 (s, 3H)
Diastéréoisomère minoritaire : 4,36 (d, 1 H), 3,96 (d, 1 H), 3,.42 (d, 1 H), 3,31 (d, 1 H), 1,51 (s, 3H)
RMN 13 C(DMSO-d6): δ ppm : 174,95; 174,63; 71,96; 70,85; 58,98; 56,73; 46,53; 45,03; 23,77; 21,96
ESI- : [(M, H) -] = 179 m/z

### Exemple 11 : Synthèse du 4-methyl-1,2-dithiolane-4-carboxylic acid 1,1-dioxide (Composé 24)

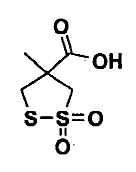

A 100mg d' Acide 4-methyl-1,2-dithiolane-4-carboxylique dans 2 ml d'acétone, sont ajoutés 2 équivalents d'eau oxygénée à 30% et 0,15 équivalent de tungstate de sodium Na2WO4. Le milieu réactionnel est agité à 20°C pendant une nuit. Après filtration et concentration sous vide, le brut est purifié sur colonne de silice pour conduire au thiosulfonate sous forme d'un solide blanc.
RMN 1 H (DMSO-d6): δ ppm 4,14 (d, 1 H), 4,05 (d, 1 H), 3,69 (d, 1 H), 3,66 (d, 1 H), 1,51 (s, 3H);
RMN 13 C (DMSO-d6): δ ppm 173,90; 65,86; 50,26; 44,58; 23,59 ESI- : [(M, H) -] = 195 m/z

### Exemple 12 : Synthèse du 4-methyl-1,2-dithiolane-4-carboxylic acid 1,1,2,2-tetraoxide (Composé 28)

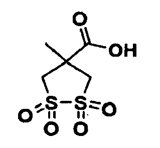

A 100mg d' Acide 4-methyl-1,2-dithiolane-4-carboxylique dans 2 ml d'acétone, sont ajoutés 4 équivalents d'eau oxygénée à 30% et 0,15 équivalent de tungstate de sodium Na₂WO₄. Le milieu réactionnel est agité à 20°C pendant une nuit. Après filtration et concentration sous vide, on obtient la disulfone cyclique sous forme d'un solide blanc.
RMN 1 H (DMSO-d6): δ ppm 3,16 (d, 2H), 2,97 (d, 2H), 1,4 (s, 3H);
RMN 13 C(DMSO-d6): δ ppm 176,31; 58,06; 43,76; 20,62
ESI- : [(M, H) -] = 227 m/z

### Exemple 13 : Synthèse du Ethyl 4-methyl-1,2-dithiolane-4-carboxylate (Composé 4)

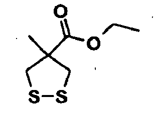

A 1g d' Acide 4-methyl-1,2-dithiolane-4-carboxylique dans 20 ml d'éthanol est ajouté de la résine sulfonique DOWEX 50 WX8 (vendue par Aldrich). Le mélange est porté au reflux pendant 24heures puis filtré et évaporé pour conduire à l'ester d'éthyle pur. RMN 1H (DMSO-d6): δ ppm 4,13 (q, 2H), 3,58 (d, 2H), 3,02 (d, 2H), 1,40 (s, 3H), 1,20 (t, 3H)
ESI+ : [(2M, Na) +] = 407 m/z

### Exemple 14 : Synthèse du Ethyl 4-methyl-1,2-dithiolane-4-carboxylate 1-oxide (Composé 25)

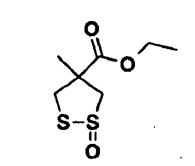

L'oxydation de l'ester d'éthyle s'effectue de la même manière que pour l'acide.
RMN 1 H (DMSO-d6): δ ppm

Diastéréoisomère majoritaire : 4,4 (d, 1H), 4,11 (q, 2H), 3,8 (d, 1H), 3,75 (d, 1H), 3,17 (d, 1H), 1.59 (s, 3H), 1.53 (t, 3H)

Diastéréoisomère minoritaire : 4,2 (d, 1H), 4,11 (q, 2H), 3,98 (d, 1H), 3,8 (d, 1H), 3,42 (d, 1H), 3.32 (d, 1H), 1.51 (s, 3H)
RMN 13 C(DMSO-d6): δ ppm 174,95, 174,63, 71,96, 70,85, 58,98, 56,73, 46,53, 45,03, 23,77, 21,96
ESI+ : [(M, Na) +] = 231 m/z ; ESI+ : [(M, Na, MeOH) +] = 263 m/z ; ESI+ : [(2M, Na) +] = 439 m/z
ESI+ : [(M, Na) +] = 231 m/z ; ESI+ : [(M, Na, MeOH) +] = 263 m/z ; ESI+ : [(2M, Na) +] = 439 m/z

### Exemple 15: Synthèse du 4-methyl-1,2-dithiolane-4-carboxamide 1-oxide (Composé 26)

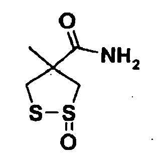

A 100 mg de 4-methyl-1,2-dithiolane-4-carboxamide dans 2 ml d'acétone, sont ajoutés 1 équivalent d'eau oxygénée à 30%. Le milieu réactionnel est agité à 20°C pendant une nuit. Après concentration sous vide et purification sur colonne de silice, on obtient le thiosulfinate sous forme d'un solide blanc mélange de 2 diastéréoisomères.
RMN 1H (DMSO-d6): δ ppm

oisomère majoritaire : 7,40 (dl, 2H), 4,31 (d, 1H), 3,78 (sl, 2H), 3,04 (d, 1H), 1,49 (s, 3H)
Diastéréoisomère minoritaire : 7,32 (dl, 2H), 4,21 (d, 1H), 3,92 (d, 1H), 3,42 (d, 1H), 3,34 (d, 1H), 1,40 (s, 3H)
ESI- : [(M, H) -] = 178 m/z

### Exemple 16 : Synthèse du 4-methyl-1,2-dithiolane-4-carboxamide 1,1-dioxide (Composé 27)

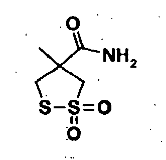

A 100mg 4-methyl-1 ,2-dithiolane-4-carboxamide dans 2 ml d'acétone, sont ajoutés 2 équivalents d'eau oxygénée à 30% et 0,15 équivalent de tungstate de sodium Na₂WO₄. Le milieu réactionnel est agité à 20°C pendant une nuit. Après filtration et concentration sous vide, le brut est purifié sur colonne de silice pour conduire au thiosulfonate sous forme d'un solide blanc.
RMN 1H (DMSO-d6): δ ppm 7,50 (dl, 2H), 4,21 (d, 1H), 4,08 (d, 1H), 3,66 (d, 1H), 3,59 (d, 1H), 1,49 (s, 3H);
ESI- : [(M, H) -] = 194 m/z

### Exemple 17: Synthèse du N-(4-hydroxy-3-methoxybenzyl)-4-methyl-1,2-dithiolane-4-carboxamide (Composé 16)

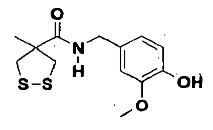

A 24,3 mmol de dithiolane acide, en solution dans 60 ml de dichorométhane, refroidi à 0°C (avec bain de glace) sont ajoutés 24,3 mmol de N-hydroxysuccinimide. Le milieu réactionnel est agité 30 min à 0°C. Une solution de 24,3 mmol de DCC dans 50ml de dichlorométhane est ajouté puis le mélange est agité à 20°C pendant 4 heures. Le milieu réactionnel est filtré et lavé puis le filtrat est évaporé à sec au rotavopor à 40°C sous vide pour conduire au 1-{[(4-methyl-1,2-dithiolan-4-yl)carbonyl]oxy}pyrrolidine-2,5-dione. (m=7g, Rdt quantitatif). A 1,58 mmol de 1-{[(4-methyl-1,2-dithiolan-4-yl)carbonyl]oxy}pyrrolidine-2,5-dione, sont ajoutés 10ml de MethylTHF, 3.16mmol de chlorhydrate de 4-(aminomethyl)-2-methoxyphenol et 3,16 mmol de triéthylamine. Après agitation une nuit, le mélange est filtré, rincé avec du MethylTHF puis évaporé. Une chromatographie flash éluant dichloromethane/ Méthanol 98/2 permet d'obtenir le composé 16 sous forme d'une huile jaune (Rdt 84%)
RMN 1H(DMSO-d6): δ ppm 1,39 (s,3H); 3,03 (d,2H); 3,56 (d, 2H), 3,63 (s, 3H, OCH3), 4,21 (d, 2H), 6,64 (dd, 1H,Ar), 6,69 (d,1H, Ar), 6.80 (d, 1H, Ar), 8,31(t, 1H, NH), 8,79 (s, 1H, OH)
ESI+ : [(M, H)+] = 300 m/z

### Exemple 18: Synthèse du N-[2-(4-hydroxy-3-methoxyphenyl)ethyl]-4-methyl-1,2-dithiolane-4-carboxamide (Composé 17)

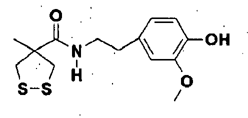

Même méthode que pour le composé 16 avec le chlorhydrate de 4-(2-aminoethyl)-2-methoxyphenol. Huile jaune Rdt=64%
RMN 1H(DMSO-d6): δ ppm 1,31 (s,3H); 2,63 (t, 2H), 2,97(d,2H); 3,24 (m,2H, NCH₂) 3,53 (d, 2H), 3,75 (s, 3H, OCH3), 6,57 (dd, 1H,Ar), 6,67 (d,1H, Ar), 674 (d, 1H, Ar), 8.86(t, 1H, NH), 8,67(s,1H, OH)
ESI+ : [(M, H)+] = 314 m/z

### Exemple 19 : Synthèse du N,N-diethyl-4-methyl-1,2-dithiolane-4-carboxamide (Composé 18)

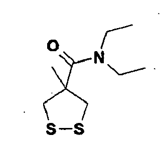

A 2.26mmol de composé 1 sont ajoutés 5 ml de dichloromethane anhydre, 0,1ml de DMF anhydre. Le mélange est refroidi à 0°C avant l'ajout de 2,7 mmol de chlorure d'oxalyl. Le mélange est agité à 20°C avant d'être ajouté à 0°C à un mélange de 2.26mmol de diéthylamine, 5ml de dichloromethane anhydre, 6.8mmol de Diisopropylethylamine. Le milieu réactionnel est agité 3h à 20°C. Lorsque la réaction est terminée, le milieu est dilué dans 50ml de dichlorométhane, puis lavé avec 2x30ml d'eau, 1x50ml de solution saturée de NH4Cl, puis séché sur Na₂SO₄ et évaporé à sec au rotavapor. Après flash chromatographie (éluant heptane/AcOEt) est isolé le composé 18 sous forme d'une huile jaune (Rdt 48%)
RMN 1H(DMSO-d6): δ ppm 1,36 (s,3H) ; 3,15 (d,2H) ; 3,50 (d, 2H), 3 3 (m, 2x2H), 1,.07(m, 2x3H)
ESI+ : [(M, H)+] = 220 m/z

### Exemple 20 : Synthèse du Methyl 2-(acetylamino)-3-{[(4-methyl-1,2-dithiolan-4-yl)carbonyl]sulfanyl}propanoate (Composé 19)

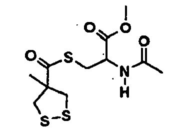

Même méthode que dans l'exemple 19 avec la N-acétylcystéine méthyle ester. Huile jaune Rdt 12%

RMN 1H(DMSO-d6): δ ppm 1,42 (s,3H) ; 1.84(s, 3H, OCH₃), 3,15 (d,2H) ; 3,.56 (d, 2H), 3,38-3,12 (dd, 2H), 3,65 (s,3H,COCH3), 8,42 (d, 1H, NH)
ESI+ : [(M, H)+] = 324 m/z

### Exemple 21: Synthèse du S-(2-hydroxyethyl) 4-methyl-1,2-dithiolane-4-carbothioate (Composé 20)

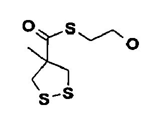

Même méthode que dans l'exemple 19 avec le sulfanyléthanol .
Huile jaune Rdt 26%
RMN 1H(DMSO-d6): δ ppm 1,43 (s,3H) ; 3,1 (d,2H) ; 3,56 (d, 2H), 2,99 (t, 2H:CH2S), 3.48 (q, 2H:CH₂OH)
ESI+ : [(M, Na)+] = 247 m/z

### Exemple 22: Synthèse du Ethyl {[(4-methyl-1,2-dithiolan-4-yl)carbonyl]sulfanyl}acetate (Composé 21)

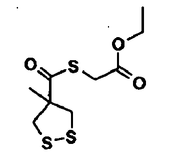

Même méthode que dans l'exemple 17 avec le thioglycolate d'éthyle. Huile incolore Rdt 7%
RMN 1H(DMSO-d6): δ ppm 1,53 (s,3H) ;1,28 (t, 3H), 2.,99 (d,2H) ; 3,65 (d, 2H),
3,71 (s, 2H, SCH2), 4,20 (q, 2H)

### Exemple 23: Synthèse du [(4-methyl-1,2-dithiolan-4-yl)carbonyl]pyrrolidine (Composé 22)

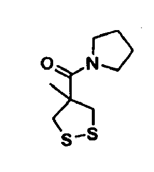

Même méthode que pour l'exemple 5-b avec la pyrrolidine
Solide jaune Rdt 38%
RMN 1H(DMSO-d6): δ ppm 1,36 (s,3H) ; 1,81 (m, 2x2H); 3,10 (d,2H) ; 3,30 (m,
2x2H); 3,58 (d, 2H),
ESI+ : [(M, H)+] = 218 m/z

### Exemple 24: Synthèse du 4-methyl-N-(1-methylethyl)-1,2-dithiolane-4-carboxamide (Composé 29)

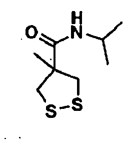

Même méthode que dans l'exemple 19 avec l'isopropylamine. Solide beige Rdt 54%
RMN 1H(DMSO-d6): δ ppm 1,06 (d, 2x3H); 1,33 (s,3H, Hc) ; 2,99 (d,2H, Hb) ; 3,56 (d, 2H, Ha); 3,88(m, 1H),
ESI+ : [(M, H)+] = 206 m/z

### Exemple 25: Synthèse du 4-methyl-N-phenyl-1,2-dithiolane-4-carboxamide (Composé 30)

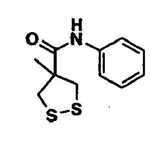

Même méthode que dans l'exemple 19 avec l'aniline.
Huile jaune Rdt 70%
RMN 1H (DMSO-d6): δ ppm 1,51 (s,3H, Hc) ; 2,99 (d,2H, Hb); 3,75 (d, 2H, Ha), 7,08 (t, 1H, Ar), 7,3( t, 2H, Ar), 7.,60(d, 2H, Ar), 9,56(s, 1h, NH)
ESI+ : [(M, H)+] = 240 m/z

### Exemple 26 - mesure de l'activité d'augmentation du taux de GSH des composés selon l'invention

L'étude a consisté à évaluer au niveau cellulaire l'effet protecteur de la molécule de référence, l'acide lipoïque, ainsi que des dérivés d'acide lipoïque selon l'invention, vis-à-vis de la déplétion du GSH intracellulaire UVJ induite.

Pour cela, des cellules HaCaT ont été exposées aux UV journaliers (UVJ). Le taux de GSH intracellulaire a ensuite été mesuré permettant alors l'évaluation de la protection éventuelle apportée par l'ajout dans le milieu de culture, des dérivés d'acide lipoïque selon l'invention.

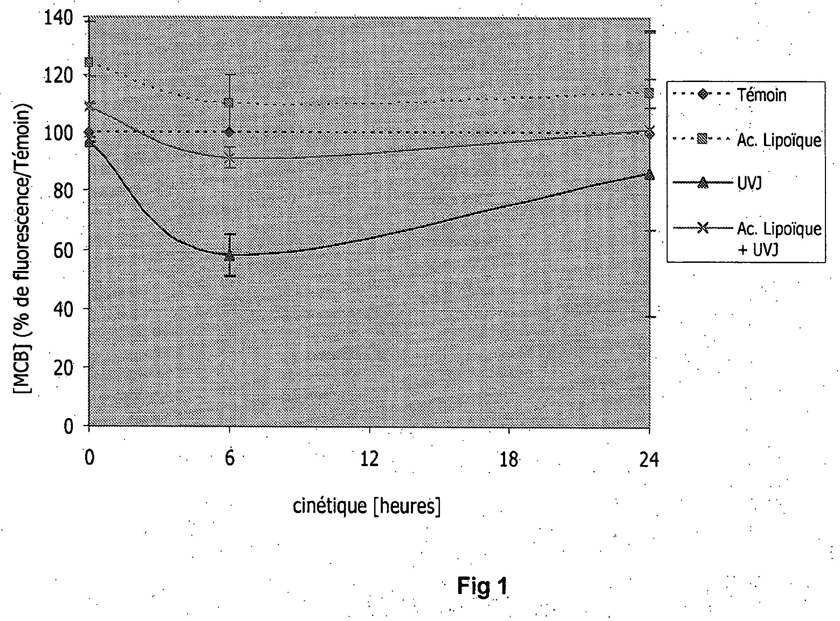

Les UV journaliers correspondent aux rayonnements d'un soleil non zénithal et pour un éclairement spectral moyen, ils simulent les rayonnements reçus par la peau d'un individu au cours d'une journée et non uniquement ceux correspondant à l'exposition au soleil zénithal. Des dispositifs permettant de reproduire ces rayonnements sont décrits dans le FR 2 863 356. La technique d'évaluation utilise une sonde fluorescente, le Monochlorobimane (MCB). Le MCB a la particularité de présenter, à l'inverse d'autres composés bimanes tel que le monobromobimane, une réactivité plus sélective vis-à-vis du glutathion : le composé fluorescent bleu (GSH-monochlorobimane) mesuré, résulte d'une réaction enzymatique catalysée par la glutathion-S-transférase. La spécificité du MCB vis-à-vis du GSH dans notre modèle kératinocytaire (lignée HaCaT) a été précédemment vérifiée. Le prétraitement des cellules avec la molécule de référence, l'acide lipoïque, pendant 24h à 1mM apporte environ 100% de protection vis-à-vis de la déplétion du GSH UVJ induite (voir la figure 1 montrant l'évaluation de l'effet protecteur de l'acide lipoïque à 1mM dans le test MCB sur HaCaT) : La mesure du taux de GSH intracellulaire est réalisée sur des kératinocytes prétraités et non prétraités avec l'acide lipoïque à 1mM et exposées aux UVJ, à t=0, t=6h, t=24h après l'exposition aux UVJ), cette protection est d'environ 83% à 500µM et d'environ 10% à 100µM.

Les composés dithiolanes de l'invention suivants ont été testés
acide 4-methyl-1,2-dithiolane-4-carboxylique (composé 1)
Octyl 4-methyl-1,2-dithiolane-4-carboxylate (composé 9)
N-heptyl-4-methyl-1,2-dithiolane-4-carboxamide (Composé 10)
4-methyl-1,2-dithiolane-4-carboxamide (Composé 2)
S-[2-(acetylamino)ethyl] 4-methyl-1,2-dithiolane-4-carbothioate (composé 13)
N-(2-hydroxyethyl)-4-methyl-1,2-dithiolane-4-carboxamide (composé 14)
Methyl 2-{[(4-methyl-1,2-dithiolan-4-yl)carbonyl]amino}-4-(methylsulfanyl)butanoate (composé 12)
4-methyl-1,2-dithiolane-4-carboxylic acid 1,1-dioxide (Composé 24)
4-methyl-1 ,2-dithiolane-4-carboxamide 1-oxide (Composé 26)
4-methyl-1,2-dithiolane-4-carboxamide 1,1-dioxide (Composé 27)

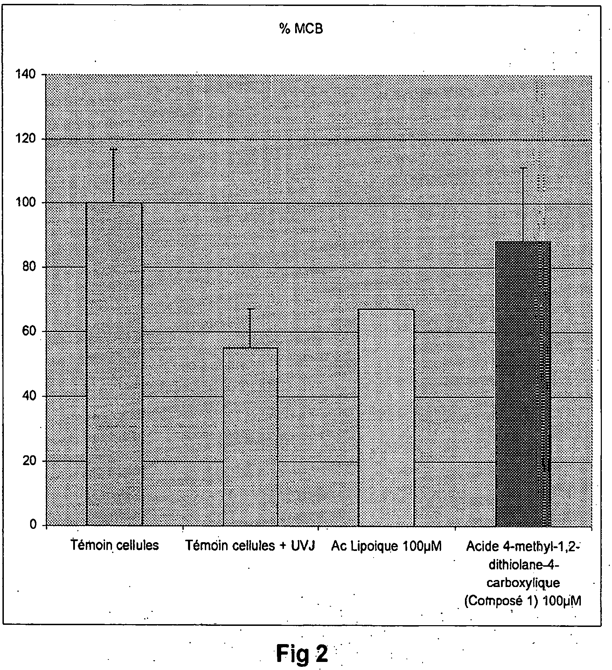

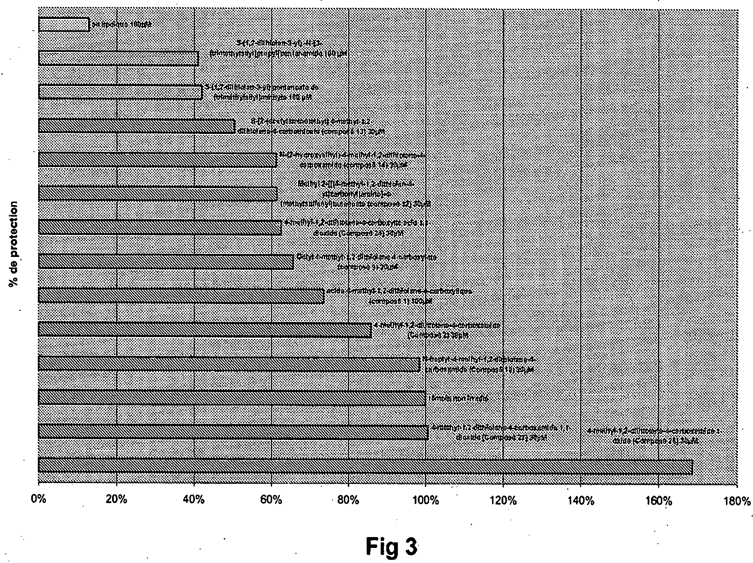

On effectue l'évaluation de l'effet protecteur du composé 1 à 100 µM et à 30 µM les composés 9, 10, 2, 13, 14, 12, 24, 26, 27 dans le Test MCB sur HaCaT: On effectue également l'évaluation de l'effet protecteur des composés dithiolanes siliciés de la demande WO2008/058999 à 100 µM dans le Test MCB sur HaCaT : le composé 5-(1,2-dithiolan-3-yl)-N-[3-(triméthylsilyl)propyl]pentanamide et le composé 5-(1,2-dithiolan-3-yl)pentanoate de (triméthylsilyl)méthyle.. La mesure du taux de GSH intracellulaire est réalisée sur des kératinocytes prétraités et non prétraités avec les actifs et exposées aux UVJ, t=6h après l'exposition aux UVJ). Les résultats sont représentés dans les graphes des figures 2 et 3.

| **Actif** | **% fluo** | **ET fluo** | **%protection** |
|---|---|---|---|
| Acide 4-methyl-1,2-dithiolane-4-carboxylique (composé 1) 100µM | 88% | 4,39% | 74% |
| 4-methyl-1,2-d ith iolane-4-carboxam ide (Composé 2) 30µM | 92% | 23,58% | 86% |
| Octyl 4-methyl-1,2-dithiolane-4-carboxylate (composé 9) 30µM | 80% | 27,21% | 66% |
| N-heptyl-4-methyl-1,2-dithiolane-4-carboxamide (Composé 10) 30µM | 99% | 30,60% | 98% |
| Methyl 2-{[(4-methyl-1,2-dithiolan-4-yl)carbonyl]amino}-4-(methylsulfanyl)butanoate (composé 12) 30µM | 78% | 12,79% | 61% |
| S-[2-(acetylamino)ethyl] 4-methyl-1,2-dithiolane-4-carbothioate (composé 13) 30µM | 71 % | 2,39% | 50% |
| N-(2-hydroxyethyl)-4-methyl-1,2-dithiolane-4-carboxamide (composé 14) 30µM | 78% | 2,79% | 61% |
| 4-methyl-1,2-dithiolane-4-carboxylic acid 1,1-dioxide (Composé 24) 30µM | 91% | 6,19% | 63% |
| 4-methyl-1,2-dithiolane-4-carboxamide 1-oxide (Composé 26) 30µM | 117% | 6,64% | 169% |
| 4-methyl-1,2-dithiolane-4-carboxamide 1-oxide (Composé 26) 30µM | 117% | 6,64% | 169% |
| 4-methyl-1,2-d ith iolane-4-carboxam ide 1,1-dioxide (Composé 27) 30µM | 100% | 3,85% | 101% |
| **acide lipoïque 100µM (hors invention)** | **63%** | **5,20%** | **13%** |
| **5-(1,2-dithiolan-3-yl) pentanoate de (triméthylsilyl)méthyle100 µM selon la demande** WO2008/058999 **(hors invention)** | **76%** | **12,00%** | **42%** |
| **5-(1,2-dithiolan-3-yl) -N-[3-(trimethylsilyl)propyl]pentanamide 100 µM selon la demande** WO2008/058999 **(hors invention)** | **75%** | **10,50%** | **41%** |

On observe que la protection apportée par ces actifs vis-à-vis de la déplétion du GSH induite par les UV Journaliers, est supérieure à la molécule de référence, l'acide lipoïque à 100 µM et ce même dès 30 µM (Voir Figures 2 et 3).

### Exemple 27 - Mesure de la protection apportée vis-à-vis des dommages à l'ADN

Les UV solaires sont capables d'engendrer de nombreux dégâts oxydatifs dans les cellules de la peau, et notamment dans l'ADN. Les acides nucléiques absorbent les photons UVB, induisant ainsi des dommages à l'ADN de façon directe. Les UVA, même s'ils ne sont pas directement absorbés par l'ADN, vont avoir une action génotoxique médiée par les espèces réactives de l'oxygène. Le test des comètes permet de quantifier les dommages à l'ADN induits par les UV (ici, les UVA) : les cassures de brin ainsi que les lésions oxydatives de l'ADN (sites alcali labiles clivés sous conditions alcalines) sont détectées.. Le principe de ce test est basé sur la capacité des fragments d'ADN dénaturés et clivés à migrer en dehors des noyaux cellulaires, sous l'influence d'un champ électrique, alors que l'ADN non endommagé reste confiné dans le noyau. Les fragments d'ADN endommagés forment une traînée derrière le noyau des cellules. Ceux-ci prennent alors l'apparence de « comètes ». La « queue » de chaque comète est d'autant plus importante et intense que les dégâts occasionnés à l'ADN sont importants. (Alapetite C. et al., 1996, Int. J. Rad. Bio. ; Klaude M. et al., 1996, Mut. Res. ; Lehmann J. et al., 1998, Mut. Res. ; Singh NP. et al., 1988, Exp. Cell Res.)

L'étude a consisté à évaluer l'effet photoprotecteur de deux molécules vis-à-vis des dommages à l'ADN UVA-induits. Pour cela, des fibroblastes humains normaux (FHN) ont été mis au contact des actifs pendant 24h. Les cellules ont ensuite été détachées, inclues dans un gel d'agarose, puis déposées sur une lame de microscope. Suite à l'exposition aux UVA (pendant 30 minutes), les cellules ont été soumises à une lyse cellulaire (de manière à ne conserver intact que le matériel génétique), à une étape de dénaturation de l'ADN, puis à une électrophorèse permettant de faire migrer l'ADN endommagé. Le taux d'ADN endommagé a été mesuré au moyen d'un microscope à fluorescence combiné à un logiciel d'analyse d'image adapté.

Le prétraitement des cellules avec le composé 1 confèrent aux fibroblastes une protection de 20% à 1µM et 30% à 10µM. Le composé 2 a été évalué dans les mêmes conditions opératoires. Il apporte une protection de 63 % à 10µM. Ces deux composés ont donc la capacité de protéger la dégradation de l'ADN induite par les UV.

| **Actifs évalués** | | | | |
|---|---|---|---|---|
| **Composé 1** | **TM moyen (dommages à l'ADN)** | **IC 95%** | **% de dommages à l'ADN** | **% de protection** |
| Témoin (UVA=30') | 11,62 | 1,32 | 100 | 0 |
| 1µm | 9.,38 | 0,77 | 80,7 | 19,3 |
| 10µM | 8,06 | 0,72 | 69,4 | 30,6 |
| **Composé 2** | **TM moyen (dommages à l'ADN)** | **IC 95%** | **% de dommages à l'ADN** | **% de protection** |
| Témoin (UVA=30') | 7,37 | 0,44 | 100 | 0 |
| 10µM | 2,73 | 0,18 | 37,1 | 62,9 |

### Exemple 28 : Composition antisolaire (émulsion huile-dans-eau)

| | |
|---|---|
| Composé de l'exemple 1, 2, 9, 10, 12,13 ou 14 | 3 g |
| Mélange d'alcool cétylstéarylique et d'alcool cétylstéarylique oxyéthyléné (33 motifs OE) 80/20 vendu par la société TENSIA sous la dénomination commerciale DEHSCONET® 390 | 3 g |
| Mélange de mono- et de distéarate de glycérol vendu sous la dénomination commerciale CERASYNTH® SD par la société ISP | 2 g |
| Alcool cétylique | 1,5 g |
| Polydiméthylsiloxane vendu sous la dénomination DC200Fluid® par la société DOW CORNING | 1,5 g |
| Glycérine | 15 g |
| Parleam = ISOPARAFFINE (6-8 MOLES D'ISOBUTYLENE) HYDROGENEE par la société NOF CORPORATION | 20 g |
| Conservateurs q.s. | |
| Eau déminéralisée qsp | 77 g |

La phase grasse contenant le composé est chauffée à environ 70 - 80 °C jusqu'à fusion complète. On ajoute ensuite sous agitation vigoureuse l'eau en une seule fois à 80°C. On maintient l'agitation pendant 10 à 15 minutes, puis on laisse refroidir sous agitation modérée jusqu'à environ 40 °C et on ajoute les conservateurs.

### Exemple 29 : Composition antisolaire (émulsion huile-dans-eau)

| | |
|---|---|
| Composé de l'exemple 1, 2, 9, 10, 12,13 ou 14 | 2 g |
| Mélange d'alcool cétylstéarylique et d'alcool cétylstéarylique oxyéthyléné (33 motifs OE) 80/20 vendu par la société TENSIA sous la dénomination commerciale DEHSCONET® 390 | 3 g |
| Mélange de mono- et de distéarate de glycérol vendu sous la dénomination commerciale CERASYNTH® SD par la société ISP | 2 g |
| Alcool cétylique | 2,5 g |
| Benzoate d'alcools en C₁₂-C₁₅ vendu sous la dénomination commerciale « FINSOLV TN » par WITCO | 20 g |
| Polydiméthylsiloxane vendu sous la dénomination DC200Fluid® par la société DOW CORNING | 1,5 g |
| Glycérine | 15 g |
| Conservateurs, parfums q.s. | |
| Eau déminéralisée qsp | 97 g |

Cette crème est préparée selon les techniques classiques de préparation d'émulsions en dissolvant le filtre dans la phase grasse contenant les émulsionnants, en chauffant cette phase grasse vers 70-80°C et en ajoutant, sous vive agitation, l'eau chauffée à la même température. On maintient l'agitation pendant 10 à 15 minutes, puis on laisse refroidir sous agitation modérée, et vers 40°C on ajoute enfin parfum et conservateur.

## Revendications

1. Utilisation cosmétique non thérapeutique d'au moins un composé dithiolane choisi parmi ceux répondant à la formule (I) 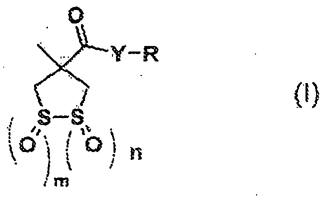 dans laquelle :
Y désigne O, NR₁ ou S
R₁ désigne un atome d'hydrogène ; un groupe hydrocarboné alkyle saturé linéaire en C₁-C₂₀ ou ramifié en C₃-C₂₀ ou insaturé en C₂-C₂₀ ; un groupe phényle éventuellement substitué par un ou plusieurs hydroxyles et/ou par une ou plusieurs radicaux alkoxy en C₁-C₈ ;
R désigne un atome d'hydrogène ; ou un groupe hydrocarboné alkyle saturé linéaire en C₁-C₂₀ ou ramifié en C₃-C₂₀ ou insaturé en C₂-C₂₀ ; ou un groupe phényle éventuellement substitué par un ou plusieurs hydroxyles et/ou par un ou
plusieurs radicaux alkoxy en C₁-C₈ ; ou un groupé alkyle saturé en C₁-C₈ possédant un substituant phényle éventuellement substitué par un ou plusieurs hydroxyles et/ou par un ou plusieurs radicaux alcoxy eh C₁-C₈.
R possède éventuellement un ou plusieurs substituants choisis parmi OR₂, SR₂, NR₂R₃, COOR₂ dans lesquels :
R₂ désigne un atome d'hydrogène ou un groupe hydrocarboné saturé linéaire en C₁-C₅ ou ramifié en C₃-C₅ ou insaturé en C₂-C₅, ou un groupe phényle
R₃ désigne un atome d'hydrogène ; un groupe hydrocarboné saturé linéaire en C₁-C₅ ou ramifié en C₃-C₅ ou insaturé en C₂-C₅, ; un groupe phényle: un groupe acétyle. Lorsque Y désigne NR₁, R et R₁ peuvent former un cycle choisi parmi pyrrolidine, pyrroline, pipéridine, pipérazine, morpholine, thiomorpholine, azépine
m = 0 ou 1 ou 2
n = 0 ou 1 ou 2
ainsi que leurs sels, leurs chélates, leurs solvates et leurs isomères optiques, dans une composition comprenant un milieu physiologiquement acceptable, dans le but de renforcer et/ou de préserver la protection antioxydante naturelle de la peau face au stress oxydatif notamment causé par les radiations UV.

2. Utilisation selon la revendication 1, pour renforcer et/ou préserver la protection antioxydante naturelle de la peau face au stress oxydatif causé par les radiations UV

3. Utilisation selon l'une des revendications 1 ou 2, où les composés de formule (I) sont choisis parmi lesquels
Y désigne S, O, NR₁
R₁ désigne un atome d'hydrogène ; un groupe hydrocarboné alkyle saturé linéaire en C₁-C₁0 ou ramifié en C₃-C₁₀ ;
R désigne un atome d'hydrogène ; un groupe hydrocarboné alkyle sature linéaire en C₁-C₂₀ ou ramifié en C₃-C₂₀ un groupe phényle éventuellement substitué par un ou plusieurs hydroxyles et/ou par un ou plusieurs radicaux alcoxy en C₁-C₃ ; un groupe alkyle saturé en C₁-C₅ possédant un substituant phényle éventuellement substitué par un ou plusieurs hydroxyles et/ou par un ou plusieurs radicaux alcoxy en C₁-C₃ ; un groupe hydrocarboné alkyle linéaire en C₁-C₅ substitué par un ou plusieurs groupes identiques ou non choisis parmi OR₂, SR₂, NR₂R₃, COOR₂ dans lesquels:
R₂ désigne un atome d'hydrogène ou un groupe hydrocarboné saturé linéaire en C₁-C₅ ou ramifié en C₃-C₅ ou insaturé en C₂-C₅,
R₃ désigne un atome d'hydrogène ; un groupe hydrocarboné saturé linéaire en C₁-C₅ ou ramifié en C₃-C₅ ; un groupe phényle ; un groupe acétyle ;
lorsque Y=NR1, R et R₁ peuvent former un cycle pyrrolidine,
n = 0 ou 1 ou 2
m = 0 ou 1 ou 2
ainsi que leurs sels d'acide ou de base, leurs chélates, leurs solvates et leurs isomères optiques.

4. Utilisation selon la revendication 3, où les composés de formule (I) sont choisis parmi lesquels
Y désigne O ou NR₁ ;
R₁ désigne un atome d'hydrogène ; un groupe hydrocarboné alkyle saturé linéaire en C₁-C₁₀ ou ramifié en C₃-C₁₀ ;
R désigne un atome d'hydrogène ; un groupe hydrocarboné alkyle saturé linéaire en C₁-C₁₀ ou ramifié en C₃-C₁₀ ; un groupe phényle éventuellement substitué par un ou plusieurs hydroxyles et/ou par un ou plusieurs radicaux methoxy ; un groupe hydrocarboné alkyle saturé en C₁-C₃ possédant un substituant phényle éventuellement substitué par un ou plusieurs hydroxyles et/ou par un ou plusieurs radicaux méthoxy ; un groupe hydrocarboné alkyle linéaire en C₁-C₄ substitué par un ou plusieurs groupes identiques ou non choisis parmi OR₂, SR₂, NR₂R₃, COOR₂ dans lesquels :
R₂ désigne un atome d'hydrogène ou un groupe hydrocarboné saturé linéaire en C₁-C₅ ou ramifié en C₃-C₅,
R₃ désigne un atome d'hydrogène ; un groupe hydrocarboné saturé linéaire en C₁-C₅ ou ramifié en C₃-C₅ ;
n = 0 ou 1 ou 2
m = 0 ou 1 ou 2
ainsi que leurs sels d'acide ou de base, leurs chélates, leurs solvates et leurs isomères optiques.

5. Utilisation selon la revendication 4, où les composés de formule (I) sont choisis parmi lesquels
Y désigne NR₁
R₁ désigne un atome d'hydrogène ; un groupe hydrocarboné alkyle saturé linéaire en C₁-C₄ ;
R désigne un atome d'hydrogène ; un groupé hydrocarboné alkyle saturé linéaire en C₁-C₁₀ ou ramifié en C₃-C₁₀ ; un groupe phényle ; un groupe alkyle saturé linéaire en C₁-C₄ substitué par un phényle substitué ou non par un ou plusieurs groupes identiques ou non choisis parmi OH, OMe ; un groupe hydrocarboné alkyle linéaire en C₁-C₄ substitué par un ou plusieurs groupes identiques ou non choisis parmi OH, NHAc, SR₂, COOR₂ avec R₂ étant un hydrogène ou un groupe alkyle linéaire en C₁-C₄ ;
n 0 ou 1 ou 2, m = 0 ou 1 ou 2
ainsi que leurs sels d'acide ou de base, leurs chélates, leurs solvates et leurs isomères optiques.

6. Utilisation selon la revendication 5, où les composés de formule (I) sont choisis parmi lesquels
Y désigne NH
R désigne un atome d'hydrogène ; un groupe hydrocarboné alkyle saturé linéaire en C₁-C₁₀ ou ramifié en C₃-C₁₀ ; un groupe phényle ; un groupe alkyle saturé linéaire en C₁-C₄ substitué par un phényle substitué ou non par un ou plusieurs groupes identiques ou non choisis parmi OH, OMe ; un groupe hydrocarboné alkyle linéaire en C₁-C₄ substitué par un ou plusieurs groupes identiques ou non choisis parmi OH, NHAc, SR₂, COOR₂ avec R₂ étant un hydrogène ou un groupe alkyle linéaire en C₁-C₄ ;
n = 0 ou 1 ou 2 ;
m = 0 ou 1 ou 2 ;
ainsi que leurs sels d'acide ou de base, leurs chélates, leurs solvates et leurs isomères optiques.

7. Utilisation selon l'une quelconque des revendications 1 à 6, où les composés de formule (I) sont choisis parmi les composés suivants :
| **N°** | **Structure** | **Nom Chimique** |
|---|---|---|
| 1 | 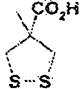 | Acide 4-methyl-1,2-dithiolane-4-carboxylique |
| 2 | 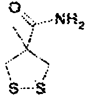 | 4-methyl-1,2-dithiolane-4-carboxamide |
| 3 | 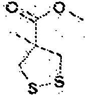 | Méthyl 4-methyl-1,2-dithiolane-4-carboxylate |
| 4 | 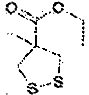 | Ethyl 4-methyl-1,2-dithiolane-4-carboxylate |
| 5 | 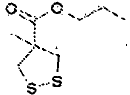 | Propyl 4-methyl-1,2-dithiolane-4-carboxylate |
| 6 | 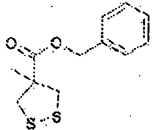 | Benzyl 4-methyl-1,2-dithiolane-4-carboxylate |
| 7 | 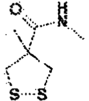 | N-methyl 4-methyl-1,2-dithiolane-4-carboxamide |
| 8 | 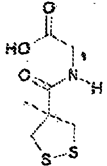 | {[(4-methyl-1,2-dithiolan-4-yl)carbonyl]amino} acetic acide |
| 9 | 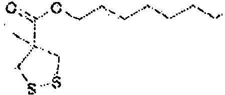 | Octyl 4-methyl-1,2-dithiolane-4-carboxylate |
| 10 | 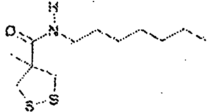 | N-heptyl-4-methyl-1,2-dithiolane-4-carboxamide |
| 11 | 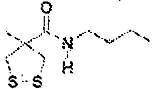 | N-butyl-4-methyl-1,2-dithiolane-4-carboxamide |
| 12 | 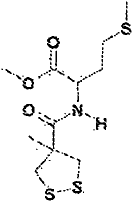 | Methyl 2-{[(4-methyl-1,2-dithiolane-4-yl)carbonyl]amino}-4-(methylsulfanyl)butanoate |
| 13 | 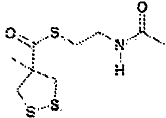 | S-[2-(acetylamino)ethyl] 4-methyl-1,2-dithiolane-4-carbothioate |
| 14 | 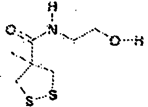 | N-(2-hydroxyethyl)-4-methyl-1,2-dithiolane-4-carboxamide |
| 15 | 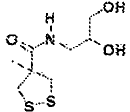 | N-(2,3-dihydroxypropyl)-4-methyl-1,2-dithiolane-4-carboxamide |
| 16 | 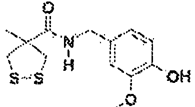 | N-(4-hydroxy-3-methoxybenzyl)-4-methyl-1,2-dithiolane-4-carboxamide |
| 17 | 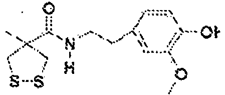 | N-[2-(4-hydroxy-3-methoxyphenyl)ethyl]-4-methyl-1,2-dithiolane-4-carboxamide |
| 18 | 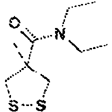 | N,N-diethyl-4-methyl-1,2-dithiolane-4-carboxamide |
| 19 | 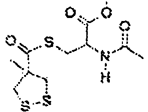 | Methyl 2-(acetylamino)-3-{[(4-methyl-1,2-dithiolan-4-yl)carbonyl]sulfanyl}propanoate |
| 20 | 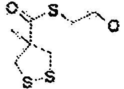 | S-(2-hydroxyethyl) 4-methyl-1,2-dithiolane-4-carbothioate |
| 21 | 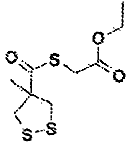 | Ethyl {[(4-methyl-1,2-dithiolan-4-yl)carbonyl]sufanyl}acetate |
| 22 | 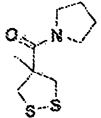 | [(4-methyl-1,2-dithiolan-4-yl)carbonyl]pyrrolidine |
| 23 | 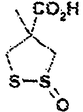 | 4-methyl-1,2-dithiolane-4-carboxylic acid 1-oxide |
| 24 | 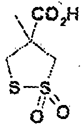 | 4-methyl-1,2-dithiolane-4-carboxylicacid 1,1-dioxide |
| 25 | 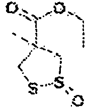 | Ethyl 4-methyl-1,2-dithiolane-4-carboxylate 1-oxide |
| 26 | 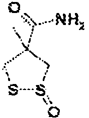 | 4-methyl-1,2-dithiolane-4-carboxamide 1-oxide |
| 27 | 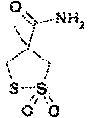 | 4-methyl-1,2-dithiolane-4-carboxamide 1,1-dioxide |
| 28 | 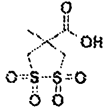 | 4-methyl-1,2-dithiolane-4-carboxylic acid 1,1,2,2-tetraoxide |
| 29 | 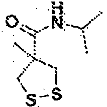 | 4-methyl-N-(1-methylethyl)-1,2-dithiolane-4-carboxamide |
| 30 | 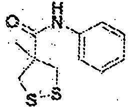 | 4-methyl-N-phenyl-1,2-dithiolane-4-carboxamide |
| 31 | 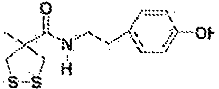 | N-[2-(4-hydroxyphenyl)ethyl]-4-methyl-1,2-dithiolane-4-carboxamide |
| 32 | 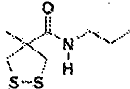 | N-propyl-4-methyl-1,2-dithiolane-4-carboxamide |
| 33 | 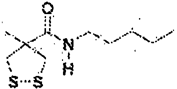 | N-pentyl-4-methyl-1,2-dithiolane-4-carboxamide |
| 34 | 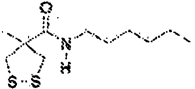 | N-hexyl-4-methyl-1,2-dithiolane-4-carboxamide |
| 35 | 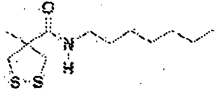 | N-octyl-4-methyl-1,2-dithiolane-4-carboxamide |
| 36 | 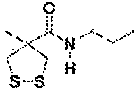 | N-propyl-4-methyl-1,2-dithiolane-4-carboxamide |
| 37 | 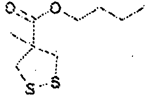 | Butyl 4-methyl-1,2-dithiolane-4-carboxylate |
| 38 | 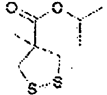 | Isopropyl 4-methyl- 1,2 - dithiolane-4-carboxylate |
| 39 | 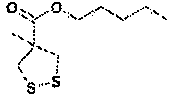 | pentyl 4-methyl-1,2-dithiolane-4-carboxylate |
| 40 | 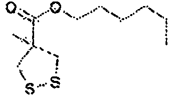 | hexyl 4-methyl-1,2-dithiolane-4-carboxylate |
| 41 | 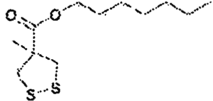 | heptyl 4-methyl-1,2-dithiolane-4-carboxylate |

8. Utilisation selon la revendication 7, où les composés de formule (I) sont choisis parmi les composés suivantes :
| **N°** | **Structure** | **Nom Chimique** |
|---|---|---|
| 1 | 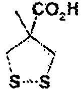 | Acide 4-methyl-1,2-dithiolane-4-carboxylique |
| 2 | 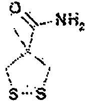 | 4-methyl-1,2-dithiolane-4-carboxamide |
| 9 | 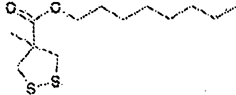 | Octyl 4-methyl-1,2-dithiolane-4-carboxylate |
| 10 | 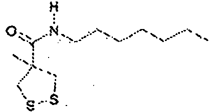 | N-heptyl-4-methyl-1,2-dithiolane-4-carboxamide |
| 26 | 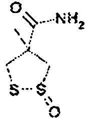 | 4-methyl-1,2-dithiolane-4-carboxamide 1-oxide |
| 27 | 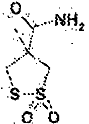 | 4-methyl-1,2-dithiolane-4-carboxamide 1,1-dioxide |

9. Utilisation selon l'une quelconque des revendications 1 à 8, dans le but de renforcer et/ou de préserver le taux de glutathion intracellulaire endogène apportant la protection antioxydante naturelle de la peau face au stress oxydatif causé par les radiations UV.

10. Utilisation selon l'une quelconque des revendications 1 à 8, dans le but de renforcer et/ou de préserver les systèmes de défense antioxydante cellulaire endogènes des cellules de la peau.

11. Utilisation selon l'une quelconque des revendications 1 à 8, dans le but de préparer la peau à une exposition scolaire.

12. Utilisation selon l'une quelconque des revendications 1 à 8, dans le but de prévenir et/ou limiter la formation de radicaux libres et/ou éliminer les radicaux libres présents dans les cellules.

13. Utilisation selon l'une quelconque des revendications 1 à 8, dans le but de prévenir et/ou traiter les signes de vieillissement cutané notamment
(i) la perte de fermeté et/ou d'élasticité et/ou de tonicité de la peau ;
(ii) l'aspect sec et rêche de la peau.
(iii) la déshydratation cutanée
(iv) l'atrophie épidermique et/ou la rugosité cutanée et/ou la sécheresse cutanée.

14. Utilisation selon l'une quelconque des revendications 1 à 8, dans le but de prévenir et/ou traiter les effets néfastes de la pollution sur la peau.

15. Composé dithiolane de formule (I) tel que défini dans les revendications 1 à 7 à l'exception des composés 1 à 8 suivants :
| **N°** | **Structure** | **Nom Chimique** |
|---|---|---|
| 1 | 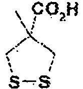 | Acide 4-methyl-1,2-dithiolane-4-carboxylique |
| 2 | 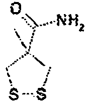 | 4-methyl-1,2-dithiolane-4-carboxamide |
| 3 | 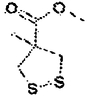 | Méthyl 4-methyl-1,2-dithiolane-4-carboxylate |
| 4 | 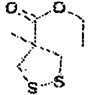 | Ethyl 4-methyl-1,2-dithiolane-4-carboxylate |
| 5 | 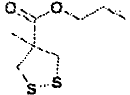 | Propyl 4-ethyl-1,2-dithiolane-4-carboxylate |
| 6 | 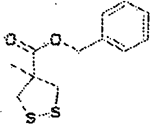 | Benzyl 4-methyl-1,2-dithiolane-4-carboxylate |
| 7 | 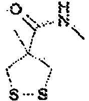 | N-methyl 4-methyl-1,2-dithiolane-4-carboxamide |
| 8 | 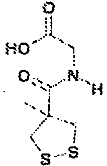 | {[(4-methyl-1,2-dithiolan-4-yl)carbonyl]amino} acetic acide |

16. Composition comprenant, dans un milieu physiologiquement acceptable, au moins un composé selon l'une quelconque des revendications 14.

## Patentansprüche

1. Nichttherapeutische kosmetische Verwendung mindestens einer Dithiolanverbindung, die aus denjenigen der Formel (I) 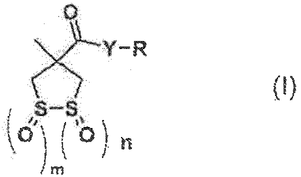 worin:
Y für O, NR₁ oder S steht;
R₁ für ein Wasserstoffatom; eine gesättigte lineare C₁-C₂₀- oder verzweigte C₃-C₂₀- oder ungesättigte C₂-C₂₀-Alkylkohlenwasserstoffgruppe oder eine Phenylgruppe, die gegebenenfalls durch eine oder mehrere Hydroxylgruppen und/oder durch einen oder mehrere C₁-C₈-Alkoxyreste substituiert ist, steht;
R für ein Wasserstoffatom oder eine gesättigte lineare C₁-C₂₀- oder verzweigte C₃-C₂₀- oder ungesättigte C₂-C₂₀-Alkylkohlenwasserstoffgruppe oder eine Phenylgruppe, die gegebenenfalls durch eine oder mehrere Hydroxylgruppen und/oder durch einen oder mehrere C₁-C₈-Alkoxyreste substituiert ist, oder eine gesättigte C₁-C₈-Alkylgruppe mit einem Phenylsubstituenten, der gegebenenfalls durch eine oder mehrere Hydroxylgruppen und/oder durch einen oder mehrere C₁-C₈-Alkoxyreste substituiert ist, steht;
R gegebenenfalls einen oder mehrere Substituenten, die aus OR₂, SR₂, NR₂R₃ und COOR₂ ausgewählt sind, aufweist, wobei:
R₂ für ein Wasserstoffatom oder eine gesättigte lineare C₁-C₅- oder verzweigte C₃-C₅- oder ungesättigte C₂-C₅-Kohlenwasserstoffgruppe oder eine Phenylgruppe steht;
R₃ für ein Wasserstoffatom; eine gesättigte lineare C₁-C₅- oder verzweigte C₃-C₅- oder ungesättigte C₂-C₅-Kohlenwasserstoffgruppe; eine Phenylgruppe oder eine Acetylgruppe steht;
R und R₁ dann, wenn Y für NR₁ steht, einen Ring, der aus Pyrrolidin, Pyrrolin, Piperidin, Piperazin, Morpholin, Thiomorpholin und Azepin ausgewählt ist, bilden können;
m = 0 oder 1 oder 2;
n = 0 oder 1 oder 2;
sowie Salzen davon, Chelaten davon, Solvaten davon und optischen Isomeren davon ausgewählt ist, in einer Zusammensetzung, die ein physiologisch unbedenkliches Medium umfasst, zur Verstärkung und/oder Bewahrung des natürlichen antioxidativen Schutzes der Haut gegen insbesondere durch UV-Strahlung verursachten oxidativen Stress.

2. Verwendung nach Anspruch 1 zur Verstärkung und/oder Bewahrung des natürlichen antioxidativen Schutzes der Haut gegen durch UV-Strahlung verursachten oxidativen Stress.

3. Verwendung nach Anspruch 1 oder 2, wobei die Verbindungen der Formel (I) unter denjenigen ausgewählt sind, worin
Y für S, O oder NR₁ steht;
R₁ für ein Wasserstoffatom oder eine gesättigte lineare C₁-C₁₀- oder verzweigte C₃-C₁₀-Alkylkohlenwasserstoffgruppe steht;
R für ein Wasserstoffatom; eine gesättigte lineare C₁-C₂₀- oder verzweigte C₃-C₂₀-Alkylkohlenwasserstoffgruppe; eine Phenylgruppe, die gegebenenfalls durch eine oder mehrere Hydroxylgruppen und/oder durch einen oder mehrere C₁-C₃-Alkoxyreste substituiert ist; eine gesättigte C₁-C₅-Alkylgruppe mit einem Phenylsubstituenten, der gegebenenfalls durch eine oder mehrere Hydroxylgruppen und/oder durch einen oder mehrere C₁-C₃-Alkoxyreste substituiert ist; oder eine lineare C₁-C₅-Alkylkohlenwasserstoffgruppe, die durch eine oder mehrere gleiche oder verschiedene Gruppen, die aus OR₂, SR₂, NR₂R₃ und COOR₂ ausgewählt sind, substituiert ist, steht, wobei:
R₂ für ein Wasserstoffatom oder eine gesättigte lineare C₁-C₅- oder verzweigte C₃-C₅- oder ungesättigte C₂-C₅-Kohlenwasserstoffgruppe steht;
R₃ für ein Wasserstoffatom; eine gesättigte lineare C₁-C₅- oder verzweigte C₃-C₅-Kohlenwasserstoffgruppe; eine Phenylgruppe oder eine Acetylgruppe steht;
R und R₁ dann, wenn Y = NR₁, einen Pyrrolidinring bilden können;
n = 0 oder 1 oder 2;
m = 0 oder 1 oder 2;
sowie Säure- oder Basensalzen davon, Chelaten davon, Solvaten davon und optischen Isomeren davon.

4. Verwendung nach Anspruch 3, wobei die Verbindungen der Formel (I) unter denjenigen ausgewählt sind, worin
Y für O oder NR₁ steht;
R₁ für ein Wasserstoffatom oder eine gesättigte lineare C₁-C₁₀- oder verzweigte C₃-C₁₀-Alkylkohlenwasserstoffgruppe steht;
R für ein Wasserstoffatom; eine gesättigte lineare C₁-C₁₀- oder verzweigte C₃-C₁₀-Alkylkohlenwasserstoffgruppe; eine Phenylgruppe, die gegebenenfalls durch eine oder mehrere Hydroxylgruppen und/oder durch einen oder mehrere Methoxyreste substituiert ist; eine gesättigte C₁-C₃-Alkylkohlenwasserstoffgruppe mit einem Phenylsubstituenten, der gegebenenfalls durch eine oder mehrere Hydroxylgruppen und/oder durch einen oder mehrere Methoxyreste substituiert ist; oder eine lineare C₁-C₄-Alkylkohlenwasserstoffgruppe, die durch eine oder mehrere gleiche oder verschiedene Gruppen, die aus OR₂, SR₂, NR₂R₃ und COOR₂ ausgewählt sind, substituiert ist, steht, wobei:
R₂ für ein Wasserstoffatom oder eine gesättigte lineare C₁-C₅- oder verzweigte C₃-C₅-Kohlenwasserstoffgruppe steht;
R₃ für ein Wasserstoffatom oder eine gesättigte lineare C₁-C₅- oder verzweigte C₃-C₅-Kohlenwasserstoffgruppe steht;
n = 0 oder 1 oder 2;
m = 0 oder 1 oder 2;
sowie Säure- oder Basensalzen davon, Chelaten davon, Solvaten davon und optischen Isomeren davon.

5. Verwendung nach Anspruch 4, wobei die Verbindungen der Formel (I) unter denjenigen ausgewählt sind, worin
Y für NR₁ steht;
R₁ für ein Wasserstoffatom oder eine gesättigte lineare C₁-C₄-Alkylkohlenwasserstoffgruppe steht;
R für ein Wasserstoffatom; eine gesättigte lineare C₁-C₁₀- oder verzweigte C₃-C₁₀-Alkylkohlenwasserstoffgruppe; eine Phenylgruppe; eine gesättigte lineare C₁-C₄-Alkylgruppe, die durch ein Phenyl, das gegebenenfalls durch eine oder mehrere gleiche oder verschiedene Gruppen, die unter OH und OMe ausgewählt sind, substituiert ist, substituiert ist; oder eine lineare C₁-C₄-Alkylkohlenwasserstoffgruppe, die durch eine oder mehrere gleiche oder verschiedene Gruppen, die aus OH, NHAc, SR₂ und COOR₂ ausgewählt sind, substituiert ist, steht, wobei R₂ für ein Wasserstoffatom oder eine lineare C₁-C₄-Alkylgruppe steht;
n = 0 oder 1 oder 2; m = 0 oder 1 oder 2;
sowie Säure- oder Basensalzen davon, Chelaten davon, Solvaten davon und optischen Isomeren davon.

6. Verwendung nach Anspruch 5, wobei die Verbindungen der Formel (I) unter denjenigen ausgewählt sind, worin
Y für NH steht;
R für ein Wasserstoffatom; eine gesättigte lineare C₁-C₁₀- oder verzweigte C₃-C₁₀-Alkylkohlenwasserstoffgruppe; eine Phenylgruppe; eine gesättigte lineare C₁-C₄-Alkylgruppe, die durch ein Phenyl, das gegebenenfalls durch eine oder mehrere gleiche oder verschiedene Gruppen, die unter OH und OMe ausgewählt sind, substituiert ist, substituiert ist; oder eine lineare C₁-C₄-Alkylkohlenwasserstoffgruppe, die durch eine oder mehrere gleiche oder verschiedene Gruppen, die aus OH, NHAc, SR₂ und COOR₂ ausgewählt sind, substituiert ist, steht, wobei R₂ für ein Wasserstoffatom oder eine lineare C₁-C₄-Alkylgruppe steht;
n = 0 oder 1 oder 2;
m = 0 oder 1 oder 2;
sowie Säure- oder Basensalzen davon, Chelaten davon, Solvaten davon und optischen Isomeren davon.

7. Verwendung nach einem der Ansprüche 1 bis 6, wobei die Verbindungen der Formel (I) aus den folgenden Verbindungen ausgewählt sind:
| **Nr.** | **Struktur** | **Chemischer Name** |
|---|---|---|
| 1 | 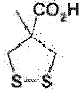 | 4-Methyl-1,2-dithiolan-4-carbonsäure |
| 2 | 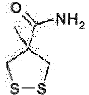 | 4-Methyl-1,2-dithiolan-4-carboxamid |
| 3 | 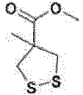 | 4-Methyl-1,2-dithiolan-4-carbonsäuremethylester |
| 4 | 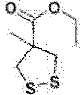 | 4-Methyl-1,2-dithiolan-4-carbonsäureethylester |
| 5 | 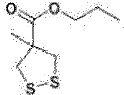 | 4-Methyl-1,2-dithiolan-4-carbonsäurepropylester |
| 6 | 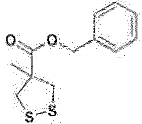 | 4-Methyl-1,2-dithiolan-4-carbonsäurebenzylester |
| 7 | 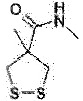 | N-Methyl-4-methyl-1,2-dithiolan-4-carboxamid |
| 8 | 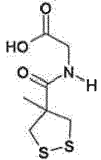 | {[(4-Methyl-1,2-dithiolan-4-yl)carbonyl]amino}essigsäure |
| 9 | 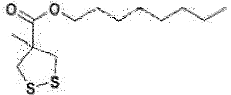 | 4-Methyl-1,2-dithiolan-4-carbonsäureoctylester |
| 10 | 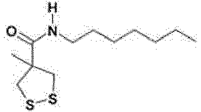 | N-Heptyl-4-methyl-1,2-dithiolan-4-carboxamid |
| 11 | 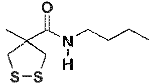 | N-Butyl-4-methyl-1,2-dithiolan-4-carboxamid |
| 12 | 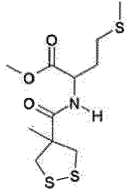 | 2-{[(4-Methyl-1,2-dithiolan-4-yl)carbonyl]amino}-4-(methylsulfanyl)butansäure-methylester |
| 13 | 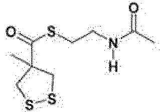 | 4-Methyl-1,2-dithiolan-4-thiocarbonsäure-S-[2-(acetylamino)ethyl]ester |
| 14 | 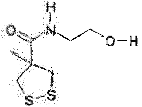 | N-(2-Hydroxyethyl)-4-methyl-1,2-dithiolan-4-carboxamid |
| 15 | 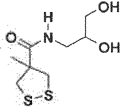 | N-(2,3-Dihydroxypropyl)-4-methyl-1,2-dithiolan-4-carboxamid |
| 16 | 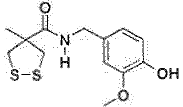 | N-(4-Hydroxy-3-methoxybenzyl)-4-methyl-1,2-dithiolan-4-carboxamid |
| 17 | 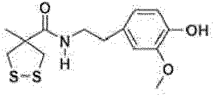 | N-[2-(4-Hydroxy-3-methoxyphenyl)ethyl]-4-methyl-1,2-dithiolan-4- |
| 18 | 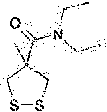 | carboxamid N,N-Diethyl-4-methyl-1,2-dithiolan-4-carboxamid |
| 19 | 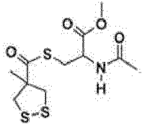 | 2-(Acetylamino)-3-{[(4-methyl-1,2-dithiolan-4-yl)-carbonyl]sulfanyl}-propansäuremethylester |
| 20 | 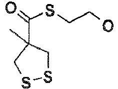 | 4-Methyl-1,2-dithiolan-4-thiocarbonsäure-S-(2-hydroxyethyl)ester |
| 21 | 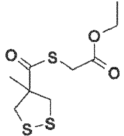 | {[(4-Methyl-1,2-dithiolan-4-yl)carbonyl]sulfanyl}-essigsäureethylester |
| 22 | 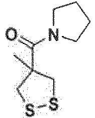 | [(4-Methyl-1,2-dithiolan-4-yl) carbonyl]pyrrolidin |
| 23 | 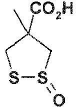 | 4-Methyl-1,2-dithiolan-4-carbonsäure-1-oxid |
| 24 | 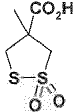 | 4-Methyl-1,2-dithiolan-4-carbonsäure-1,1-dioxid |
| 25 | 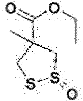 | 4-Methyl-1,2-dithiolan-4-carbonsäureethylester-1-oxid |
| 26 | 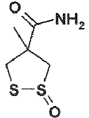 | 4-Methyl-1,2-dithiolan-4-carboxamid-1-oxid |
| 27 | 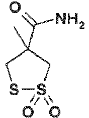 | 4-Methyl-1,2-dithiolan-4-carboxamid-1,1-dioxid |
| 28 | 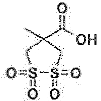 | 4-Methyl-1,2-dithiolan-4-carbonsäure-1,1,2,2-tetraoxid |
| 29 | 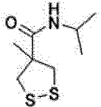 | 4-Methyl-N-(1-methylethyl)-1,2-dithiolan-4-carboxamid |
| 30 | 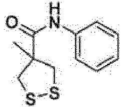 | 4-Methyl-N-phenyl-1,2-dithiolan-4-carboxamid |
| 31 | 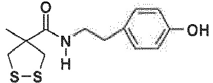 | N-[2-(4-Hydroxyphenyl)ethyl]-4-methyl-1,2-dithiolan-4-carboxamid |
| 32 | 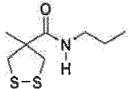 | N-Propyl-4-methyl-1,2-dithiolan-4-carboxamid |
| 33 | 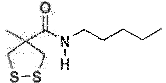 | N-Pentyl-4-methyl-1,2-dithiolan-4-carboxamid |
| 34 | 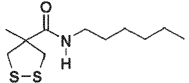 | N-Hexyl-4-methyl-1,2-dithiolan-4-carboxamid |
| 35 | 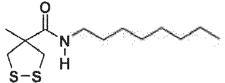 | N-Octyl-4-methyl-1,2-dithiolan-4-carboxamid |
| 36 | 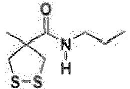 | N-Propyl-4-methyl-1,2-dithiolan-4-carboxamid |
| 37 | 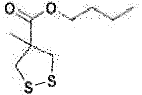 | 4-Methyl-1,2-dithiolan-4-carbonsäurebutylester |
| 38 | 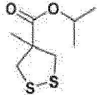 | 4-Methyl-1,2-dithiolan-4-carbonsäureisopropylester |
| 39 | 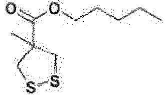 | 4-Methyl-1,2-dithiolan-4-carbonsäurepentylester |
| 40 | 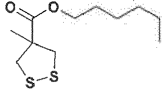 | 4-Methyl-1,2-dithiolan-4-carbonsäurehexylester |
| 41 | 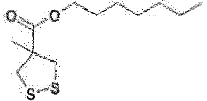 | 4-Methyl-1,2-dithiolan-4-carbonsäureheptylester |

8. Verwendung nach Anspruch 7, wobei die Verbindungen der Formel (I) aus den folgenden Verbindungen ausgewählt sind:
| **Nr.** | **Struktur** | **Chemischer Name** |
|---|---|---|
| 1 | 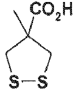 | 4-Methyl-1,2-dithiolan-4-carbonsäure |
| 2 | 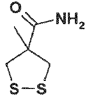 | 4-Methyl-1,2-dithiolan-4-carboxamid |
| 9 | 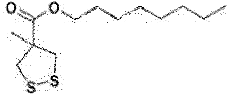 | 4-Methyl-1,2-dithiolan-4-carbonsäureoctylester |
| 10 | 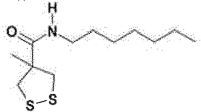 | N-Heptyl-4-methyl-1,2-dithiolan-4-carboxamid |
| 26 | 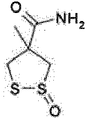 | 4-Methyl-1,2-dithiolan-4-carboxamid-1-oxid |
| 27 | 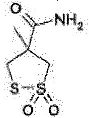 | 4-Methyl-1,2-dithiolan-4-carboxamid-1,1-dioxid |

9. Verwendung nach einem der Ansprüche 1 bis 8 zur Verstärkung und/oder Bewahrung des Niveaus von endogenem intrazellulärem Glutathion, das für den natürlichen antioxidativen Schutz der Haut gegen durch UV-Strahlung verursachten oxidativen Stress sorgt.

10. Verwendung nach einem der Ansprüche 1 bis 8 zur Verstärkung und/oder Bewahrung der endogenen Systeme zur zellulären antioxidativen Verteidigung von Hautzellen.

11. Verwendung nach einem der Ansprüche 1 bis 8 zur Vorbereitung der Haut auf Einwirkung von Sonnenlicht.

12. Verwendung nach einem der Ansprüche 1 bis 8 zur Prävention und/oder Einschränkung der Bildung von Radikalen und/oder Eliminierung von Radikalen, die in Zellen vorliegen.

13. Verwendung nach einem der Ansprüche 1 bis 8 zur Prävention und/oder Behandlung von Anzeichen für Hautalterung, insbesondere
(i) Verlust der Festigkeit und/oder Elastizität und/oder Spannkraft der Haut;
(ii) trockenem und rauem Aussehen der Haut;
(iii) Dehydratisierung der Haut;
(iv) epidermaler Atrophie und/oder Rauheit der Haut und/oder Trockenheit der Haut.

14. Verwendung nach einem der Ansprüche 1 bis 8 zur Prävention und/oder Behandlung von schädlichen Auswirkungen von Umweltverschmutzung auf die Haut.

15. Dithiolanverbindung der Formel (I) gemäß den Ansprüchen 1 bis 7 mit Ausnahme der folgenden Verbindungen 1 bis 8:
| **Nr.** | **Struktur** | **Chemischer Name** |
|---|---|---|
| 1 | 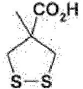 | 4-Methyl-1,2-dithiolan-4-carbonsäure |
| 2 | 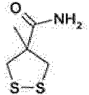 | 4-Methyl-1,2-dithiolan-4-carboxamid |
| 3 | 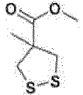 | 4-Methyl-1,2-dithiolan-4-carbonsäuremethylester |
| 4 | 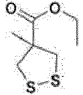 | 4-Methyl-1,2-dithiolan-4-carbonsäureethylester |
| 5 | 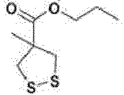 | 4-Methyl-1,2-dithiolan-4-carbonsäurepropylester |
| 6 | 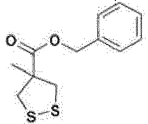 | 4-Methyl-1,2-dithiolan-4-carbonsäurebenzylester |
| 7 | 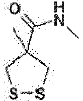 | N-Methyl-4-methyl-1,2-dithiolan-4-carboxamid |
| 8 | 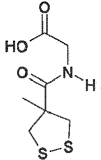 | {[(4-Methyl-1,2-dithiolan-4-yl)carbonyl]amino}essigsäure |

16. Zusammensetzung, die in einem physiologisch unbedenklichen Medium mindestens eine Verbindung nach einem der vorhergehenden Ansprüche 15 umfasst.

## Claims

1. Non therapeutic cosmetic use of at least one dithiolane compound chosen from those corresponding to formula (I): 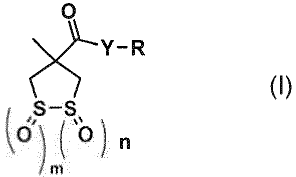 in which:
Y denotes O, NR₁ or S;
R₁ denotes a hydrogen atom; a saturated linear C₁-C₂₀ or branched C₃-C₂₀ or unsaturated C₂-C₂₀ alkyl hydrocarbon-based group; a phenyl group optionally substituted with one or more hydroxyls and/or with one or more C₁-C₈ alkoxy radicals;
R denotes a hydrogen atom; a saturated linear C₁-C₂₀ or branched C₃-C₂₀ or unsaturated C₂-C₂₀ alkyl hydrocarbon-based group; a phenyl group optionally substituted with one or more hydroxyls and/or with one or more C₁-C₈ alkoxy radicals; or a saturated C₁-C₈ alkyl group containing a phenyl substituent optionally substituted with one or more hydroxyls and/or with one or more C₁-C₈ alkoxy radicals;
R optionally bears one or more substituents chosen from OR₂, SR₂, NR₂R₃ and COOR₂ in which:
R₂ denotes a hydrogen atom or a saturated linear C₁-C₅ or branched C₃-C₅ or unsaturated C₂-C₅ hydrocarbon-based group, or a phenyl group;
R₃ denotes a hydrogen atom; a saturated linear C₁-C₅ or branched C₃-C₅ or unsaturated C₂-C₅ hydrocarbon-based group; a phenyl group; an acetyl group. When Y denotes NR₁, R and R₁ may form a ring chosen from pyrrolidine, pyrroline, piperazine, morpholine, thiomorpholine and azepine;
m = 0 or 1 or 2;
n = 0 or 1 or 2;
and also the salts thereof, chelates thereof, solvates thereof and optical isomers thereof, in a composition comprising a physiologically acceptable medium, for the purpose of reinforcing and/or preserving the natural antioxidant protection of the skin against oxidative stress caused especially by UV radiation.

2. Use according to Claim 1, in order to reinforce and/or preserve the natural antioxidant protection of the skin against oxidative stress caused by UV radiation.

3. Use according to either of Claim 1 and 2, in which the compounds of formula (I) are chosen from those for which:
Y denotes S, O or NR₁;
R₁ denotes a hydrogen atom; a saturated linear C₁-C₁₀ or branched C₃-C₁₀ alkyl hydrocarbon-based group;
R denotes a hydrogen atom; a saturated linear C₁-C₂₀ or branched C₃-C₂₀ alkyl hydrocarbon-based group; a phenyl group optionally substituted with one or more hydroxyls and/or with one or more C₁-C₃ alkoxy radicals; a saturated C₁-C₅ alkyl group bearing a phenyl substituent optionally substituted with one or more hydroxyls and/or with one or more C₁-C₃ alkoxy radicals; a linear C₁-C₅ alkyl hydrocarbon-based group substituted with one or more identical or different groups chosen from OR₂, SR₂, NR₂R₃ and COOR₂ in which:
R₂ denotes a hydrogen atom or a saturated linear C₁-C₅ or branched C₃-C₅ or unsaturated C₂-C₅ hydrocarbon-based group;
R₃ denotes a hydrogen atom; a saturated linear C₁-C₅ or branched C₃-C₅ hydrocarbon-based group; a phenyl group; an acetyl group;
when Y = NR₁, R and R₁ may form a pyrrolidine ring;
n = 0 or 1 or 2;
m = 0 or 1 or 2;
and also the acid or base salts thereof, chelates thereof, solvates thereof and optical isomers thereof.

4. Use according to Claim 3, in which the compounds of formula (I) are chosen from those for which:
Y denotes O or NR₁;
R₁ denotes a hydrogen atom; a saturated linear C₁-C₁₀ or branched C₃-C₁₀ alkyl hydrocarbon-based group;
R denotes a hydrogen atom; a saturated linear C₁-C₁₀ or branched C₃-C₁₀ alkyl hydrocarbon-based group; a phenyl group optionally substituted with one or more hydroxyls and/or with one or more methoxy radicals; a saturated C₁-C₃ alkyl hydrocarbon-based group bearing a phenyl substituent optionally substituted with one or more hydroxyls and/or with one or more methoxy radicals; a linear C₁-C₄ alkyl hydrocarbon-based group substituted with one or more identical or different groups chosen from OR₂, SR₂, NR₂R₃ and COOR₂ in which:
R₂ denotes a hydrogen atom or a saturated linear C₁-C₅ or branched C₃-C₅ hydrocarbon-based group;
R₃ denotes a hydrogen atom; a saturated linear C₁-C₅ or branched C₃-C₅ hydrocarbon-based group;
n = 0 or 1 or 2;
m = 0 or 1 or 2;
and also the acid or base salts thereof, chelates thereof, solvates thereof and optical isomers thereof.

5. Use according to Claim 4, in which the compounds of formula (I) are chosen from those for which:
Y denotes NR₁;
R₁ denotes a hydrogen atom; a saturated linear C₁-C₄ alkyl hydrocarbon-based group;
R denotes a hydrogen atom; a saturated linear C₁-C₁₀ or branched C₁-C₁₀ alkyl hydrocarbon-based group; a phenyl group; a saturated linear C₁-C₄ alkyl group substituted with a phenyl optionally substituted with one or more identical or different groups chosen from OH and OMe; a linear C₁-C₄ alkyl hydrocarbon-based group substituted with one or more identical or different groups chosen from OH, NHAc, SR₂ and COOR₂ with R₂ being a hydrogen or a linear C₁-C₄ alkyl group;
n = 0 or 1 or 2, m = 0 or 1 or 2 ;
and also the acid or base salts thereof, chelates thereof, solvates thereof and optical isomers thereof.

6. Use according to Claim 5, in which the compounds of formula (I) are chosen from those for which:
Y denotes NH;
R denotes a hydrogen atom; a saturated linear C₁-C₁₀ or branched C₃-C₁₀ alkyl hydrocarbon-based group; a phenyl group; a saturated linear C₁-C₄ alkyl group substituted with a phenyl optionally substituted with one or more identical or different groups chosen from OH and OMe; a linear C₁-C₄ alkyl hydrocarbon-based group substituted with one or more identical or different groups chosen from OH, NHAc, SR₂ and COOR₂ with R₂ being a hydrogen or a linear C₁-C₄ alkyl group;
n = 0 or 1 or 2 ;
m = 0 or 1 or 2 ;
and also the acid or base salts thereof, chelates thereof, solvates thereof and optical isomers thereof.

7. Use according to any one of Claims 1 to 6, in which the compounds of formula (I) are chosen from the following compounds:
| **No.** | **Structure** | **Chemical name** |
|---|---|---|
| 1 | 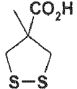 | 4-methyl-1,2-dithiolane-4-carboxylic acid |
| 2 | 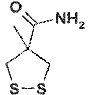 | 4-methyl-1,2-dithiolane-4-carboxamide |
| 3 | 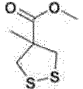 | methyl 4-methyl-1,2-dithiolane-4-carboxylate |
| 4 | 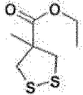 | ethyl 4-methyl-1,2-dithiolane-4-carboxylate |
| 5 | 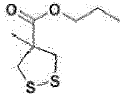 | propyl 4-methyl-1,2-dithiolane-4-carboxylate |
| 6 | 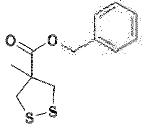 | benzyl 4-methyl-1,2-dithiolane-4-carboxylate |
| 7 | 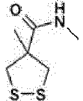 | N-methyl-4-methyl-1,2-dithiolane-4-carboxamide |
| 8 | 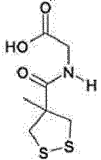 | {[(4-methyl-1,2-dithiolan-4-yl)-carbonyl]amino}-acetic acid |
| 9 | 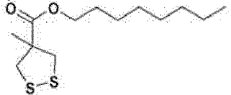 | octyl 4-methyl-1,2-dithiolane-4-carboxylate |
| 10 | 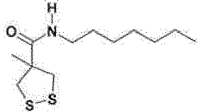 | N-heptyl-4-methyl-1,2-dithiolane-4-carboxamide |
| 11 | 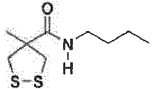 | N-butyl-4-methyl-1,2-dithiolane-4-carboxamide |
| 12 | 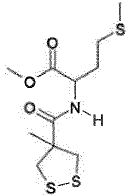 | methyl 2-{[(4-methyl-1,2-dithiolan-4-yl)-carbonyl]amino}-4-(methylsulfanyl)-butanoate |
| 13 | 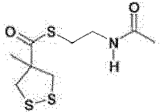 | S-[2-(acetylamino)ethyl] 4-methyl-1,2-dithiolane-4-carbothioate |
| 14 | 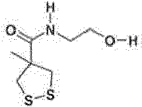 | N-(2-hydroxy-ethyl)-4-methyl-1,2-dithiolane-4-carboxamide |
| 15 | 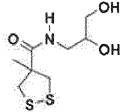 | N-(2,3-dihydroxy-propyl)-4-methyl-1,2-dithiolane-4-carboxamide |
| 16 | 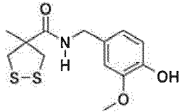 | N-(4-hydroxy-3-methoxybenzyl)-4-methyl-1,2-dithiolane-4-carboxamide |
| 17 | 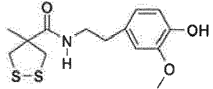 | N-[2-(4-hydroxy-3-methoxyphenyl)ethyl ]-4-methyl-1,2-dithiolane-4-carboxamide |
| 18 | 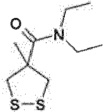 | N,N-diethyl-4-methyl-1,2-dithiolane-4-carboxamide |
| 19 | 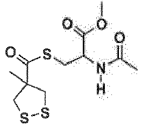 | methyl 2-(acetylamino)-3-{[(4-methyl-1,2-dithiolan-4-yl)-carbonyl]sulfanyl}- |
| 20 | 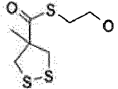 | propanoate S-(2-hydroxyethyl) 4-methyl-1,2-dithiolane-4-carbothioate |
| 21 | 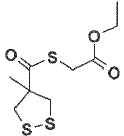 | ethyl {[(4-methyl-1,2-dithiolan-4-yl)carbonyl]sulfanyl}acetate |
| 22 | 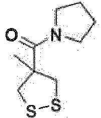 | [(4-methyl-1,2-dithiolan-4-yl)-carbonyl]pyrrolidine |
| 23 | 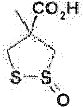 | 4-ethyl-1,2-dithiolane-4-carboxylic acid 1-oxide |
| 24 | 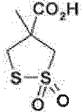 | 4-methyl-1,2-dithiolane-4-carboxylic acid 1,1-dioxide |
| 25 | 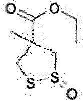 | ethyl 4-methyl-1,2-dithiolane-4-carboxylate 1-oxide |
| 26 | 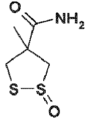 | 4-methyl-1,2-dithiolane-4-carboxamide 1-oxide |
| 27 | 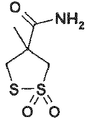 | 4-methyl-1,2-dithiolane-4-carboxamide 1,1- |
| 28 | 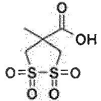 | dioxide 4-methyl-1,2-dithiolane-4-carboxylic acid 1,1,2,2-tetroxide |
| 29 | 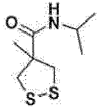 | 4-methyl-N-(1-methylethyl)-1,2-dithiolane-4-carboxamide |
| 30 | 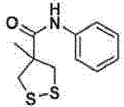 | 4-methyl-N-phenyl-1,2-dithiolane-4-carboxamide |
| 31 | 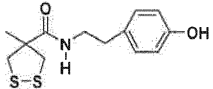 | N-[2-(4-hydroxy-phenyl)ethyl]-4-methyl-1,2-dithiol- |
| 32 | 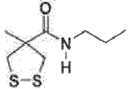 | ane-4-carboxamide N-propyl-4-methyl-1,2-dithiolane-4-carboxamide |
| 33 | 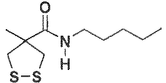 | N-pentyl-4-methyl-1,2-dithiolane-4-carboxamide |
| 34 | 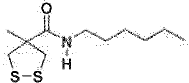 | N-hexyl-4-methyl-1,2-dithiolane-4-carboxamide |
| 35 | 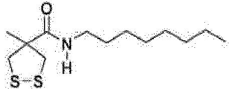 | N-octyl-4-methyl-1,2-dithiolane-4-carboxamide |
| 36 | 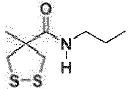 | N-propyl-4-methyl-1,2-dithiolane-4-carboxamide |
| 37 | 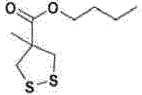 | butyl 4-methyl-1,2-dithiolane-4-carboxylate |
| 38 | 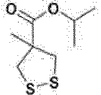 | isopropyl 4-methyl-1,2-dithiolane-4-carboxylate |
| 39 | 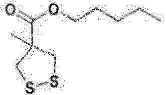 | pentyl 4-methyl-1,2-dithiolane-4-carboxylate |
| 40 | 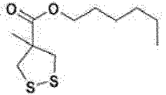 | hexyl 4-methyl-1,2-dithiolane-4-carboxylate |
| 41 | 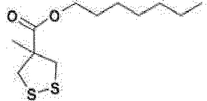 | heptyl 4-methyl-1,2-dithiolane-4-carboxylate |

8. Use according to Claim 7, in which the compounds of formula (I) are chosen from the following compounds:
| **No.** | **Structure** | **Chemical name** |
|---|---|---|
| 1 | 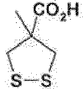 | 4-methyl-1,2-dithiolane-4-carboxylic acid |
| 2 | 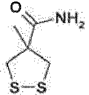 | 4-methyl-1,2-dithiolane-4-carboxamide |
| 9 | 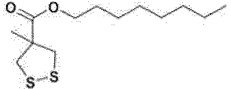 | octyl 4-methyl-1,2-dithiolane-4-carboxylate |
| 10 | 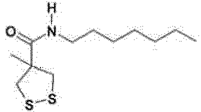 | N-heptyl-4-methyl-1,2-dithiolane-4-carboxamide |
| 26 | 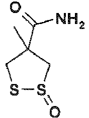 | 4-methyl-1,2-dithiolane-4-carboxamide 1-oxide |
| 27 | 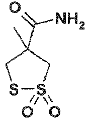 | 4-methyl-1,2-dithiolane-4-carboxamide 1,1-dioxide |

9. Use according to any one of Claims 1 to 8, for the purpose of reinforcing and/or preserving the level of endogenous intracellular glutathione that gives the skin natural antioxidant protection against oxidative stress caused especially by UV radiation.

10. Use according to any one of Claims 1 to 8, for the purpose of reinforcing and/or preserving cellular antioxidant defence systems, in particular the endogenous antioxidant defence systems of skin cells.

11. Use according to any one of Claims 1 to 8, for the purpose of preparing skin for exposure to sunlight.

12. Use according to any one of Claims 1 to 8, for the purpose of preventing and/or limiting the formation of free radicals and/or removing free radicals present in cells.

13. Use according to any one of Claims 1 to 8, for the purpose of preventing and/or treating the signs of ageing of the skin, especially:
(i) loss of firmness and/or elasticity and/or tonicity of the skin;
(ii) the dry and coarse appearance of the skin;
(iii) skin dehydration;
(iv) epidermal atrophy and/or skin roughness and/or skin dryness.

14. Use according to any one of Claims 1 to 8, for the purpose of preventing and/or treating the harmful effects of pollution on the skin.

15. Dithiolane compound of formula (I) as defined in Claims 1 to 7, with the exception of compounds 1 to 8 below:
| **No.** | **Structure** | **Chemical name** |
|---|---|---|
| 1 | 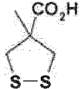 | 4-methyl-1,2-dithiolane-4-carboxylic acid |
| 2 | 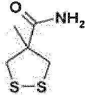 | 4-methyl-1,2-dithiolane-4-carboxamide |
| 3 | 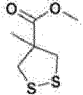 | methyl 4-methyl-1,2-dithiolane-4-carboxylate |
| 4 | 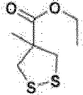 | ethyl 4-methyl-1,2-dithiolane-4-carboxylate |
| 5 | 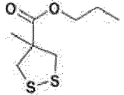 | propyl 4-methyl-1,2-dithiolane-4-carboxylate |
| 6 | 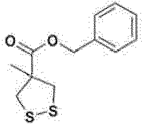 | benzyl 4-methyl-1,2-dithiolane-4-carboxylate |
| 7 | 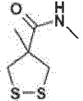 | N-methyl-4-methyl-1,2-dithiolane-4-carboxamide |
| 8 | 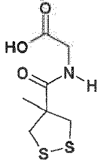 | {[(4-methyl-1,2-dithiolan-4-yl)-carbonyl]amino}-acetic acid |

16. Composition comprising, in a physiologically acceptable medium, at least one compound according to any one of Claims 15.
